(19) Europäisches Patentamt
European Patent Office
Office européen des brevets

(11) EP 1 710 240 B1

(12) FASCICULE DE BREVET EUROPEEN

(45) Date de publication et mention de la délivrance du brevet:
**05.12.2007 Bulletin 2007/49**

(21) Numéro de dépôt: **06290575.7**

(22) Date de dépôt: **07.04.2006**

(51) Int Cl.:
*C07D 313/08* (2006.01) *C07D 211/22* (2006.01)
*C07D 215/42* (2006.01) *C07D 295/08* (2006.01)
*C07D 243/08* (2006.01) *C07D 311/68* (2006.01)
*C07D 405/04* (2006.01) *C07D 295/14* (2006.01)
*C07D 319/20* (2006.01) *A61K 31/496* (2006.01)
*A61K 31/453* (2006.01) *A61K 31/5377* (2006.01)
*A61K 31/551* (2006.01) *A61K 31/4465* (2006.01)
*A61P 25/24* (2006.01) *C07D 213/81* (2006.01)
*C07D 249/12* (2006.01)

# (54) Dérives de pipérazine et leur utilisation comme inhibiteurs de recapture de la sérotonine ou comme antagonistes de neurokinine

Piperazinderivate und ihre Verwendung als Serotonin-Wiederaufnahme-Inhibitoren oder als Neurokinin-Antagonisten

Piperazine derivatives and their use as serotonin reuptake inhibitors or as neurokinin antagonists

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **08.04.2005 FR 0503512**

(43) Date de publication de la demande:
**11.10.2006 Bulletin 2006/41**

(73) Titulaire: **Les Laboratoires Servier
92415 Courbevoie Cedex (FR)**

(72) Inventeurs:
- **Peglion, Jean-Louis
78110 Le Vesinet (FR)**
- **Dessinges, Aimée
92500 Rueil-Malmaison (FR)**
- **Goument, Bertrand
78220 Viroflay (FR)**
- **Millan, Mark
78230 Le Pecq (FR)**
- **Mannoury La Cour, Clotilde
75014 Paris (FR)**

(74) Mandataire: **Giudicelli, Cathy
Les Laboratoires Servier
Direction Brevets
12, Place de la Défense
F-92415 Courbevoie (FR)**

(56) Documents cités:
**US-A- 6 136 824**

- **RYCKMANS T ET AL: "Dual NK(1) antagonists - Serotonin reuptake inhibitors as potential antidepressants. Part 2: SAR and activity of benzyloxyphenethyl piperazine derivatives" BIOORGANIC & MEDICINAL CHEMISTRY LETTERS, vol. 12, no. 21, 4 novembre 2002 (2002-11-04), pages 3195-3198, XP002317322**
- **BHANDARI K ET AL: "Synthesis of 1-amino-1,2,3,4-tetrahydro-naphthalen- 2-ols via epoxide ring opening as possible antidepressant and anorexigenic agents" INDIAN JOURNAL OF CHEMISTRY, SECTION B, vol. 39B, juin 2000 (2000-06), pages 468-471, XP008059559**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 710 240 B1

## Description

La présente invention concerne des dérivés de pipérazine, leur procédé de préparation et les compositions pharmaceutiques qui les contiennent, ainsi que leur utilisation en tant qu'inhibiteurs de recapture de la sérotonine.

A ce titre, ils sont utiles dans le traitement des états dépressifs (Goodnick and Goldstein, J Psychopharmacol 1998, 12 (Suppl B):S55-S87; Cheer and Goa, Drugs 2001, 61:81-110; MacQueen et al, CNS Drug Rev 2001, 7:1-24; Wagstaff et al, Drugs 2002, 62:655-703), des états anxieux tels que l'anxiété généralisée, les attaques de paniques et les phobies (Feighner, J Clin Psychiatry 1999, 60 (Suppl 22):18-22; Bakker et al, Int clin Psychopharmacol 2000, 15 (Suppl 2):S25-S30; Davidson, Int Clin Psychopharmacol 2000, 15(suppl 1):S13-S17; Schneier, J Clin Psychiatry 2001, 62(Suppl 1):43-48), l'impact néfaste du stress tantôt psychologique (Marona-Lewicka and Nichols, Stress 1997, 2: 91-100; Mar et al, Pharmacol Biochem Behav 2002, 73:703-712; Will et al, Mol Psychiatry 2003, 8:925-932; Ballenger, J Clin Psychiatry 2004, 65:1696-1707), tantôt neurotoxique (Malberg and Duman, Neuropsychopharmacology 2003, 28:1562-1571; Santarelli et al, Science 2003, 301:805-809; Czeh et al, Neuropsychopharmacology 2005, 30:67-79; Malberg and Schechter, Curr Pharm Des 2005, 11:145-155), des états impulsifs tels que les "TOC", ou troubles de comportement obsessionnel-compulsif (Njung'e and Handley, Br J Pharmacol 1991, 104:105-112; Ichimaru et al, Jpn J Pharmacol 1995, 68:65-70; Pigott and Seay, J Clin Psychiatry 1999, 60:101-106; Vythilingum et al, Int Clin Psychopharmacol 2000, 15 (Suppl 2)S7-S13), des états agressifs (Knutson et al, Am J Psychiatry 1998, 155:373-379; Lanctot et al, Neuropsychopharmacology 2002, 27:646-654; New et al, Psychopharmacology 2004, 176:451-458), de l'obésité et des troubles de l'appétit tels que la boulimie (Proietto et al, Expert Opin Investig Drugs 2000, 9:1317-1326; Ljung et al, J Intern Med 2001, 250:219-224; Appolinario et al, CNS Drugs 2004, 18:629-651; Appolinario and McElroy, Curr Drug Targets 2004, 5:301-307), des états douloureux (Aragona et al, Eur J Pain 2005, 9:33-38; Millan et al, Neuropharmacology 2002, 42:677-684; Duman et al, J Pharmacol Sci 2004, 94:161-165; Otsuka et al, J Anesth 2004, 15:154-158); en rapport avec ces éléments, des troubles de comportements et de dégénération neuronale liées à la démence et d'autres maladies du vieillissement (Lyketos et al, Am JPsychiatry 2000, 157:1686-1689; Lanctot et al, J Neuropsyhiatry Clin Neurosci 2001, 13:5-21; Lanctot et al, Neuropsychopharmacology 2002, 27:646-654; Pollock et al, Am J Psychiatry 2002, 159:460-465).

De plus, la plupart des composés de la présente invention sont également actifs comme antagonistes de neurokinine $NK_1$.

A ce titre, ils sont également utiles dans le traitement des états dépressifs (Rupniak et al, Behav Pharmacol 2001, 12: 497-508; Rupniak et al, Neuropharmacology 2003, 44:516-523; Kramer et al, Neuropsychopharmacology 2004, 29: 385-392; Dableh et al, Eur J Pharmacol 2005, 507:99-105), des états anxieux tels que l'anxiété généralisée, les attaques de paniques et les phobies (Rupniak et al, Behav Pharmacol 2001, 12:497-508; Santarelli et al, Proc Natl Acad Sci USA 2001, 98:1912-1927; Varty et al, Neuropsychopharmacology 2002, 27:371-379; Rupniak and Kramer, Neuropsychopharmacology 2002, 13:169-177), de l'impact néfaste du stress, tantôt psychologique (Ballard et al, Eur J Pharmacol 2001, 412:255-264; Rupniak and Kramer, Neuropsychopharmacology 2002, 13:169-177; Spooren et al, Eur J Pharmacol 2002, 435:161-170; Steinberg et al, J Pharmacol Exp Ther 2002, 303:1180-1188), tantôt neurotoxique (Van der Hart et al, Mol Psychiatry 2002, 7:933-941; Morcuende et al, Eur J Neurosci 2003, 18:1828-1836; Guest et al, Brain Res 2004, 1002:1-10; Czeh et al, Neuropsychopharmacology 2005, 30:67-79), des états impulsifs tels que les troubles du comportement obsessionnel-compulsif (Culman et al, Br J Pharmacol 1995, 114:1310-1316; Tschöpe et al, Br J Pharmacol 1992, 107:750-755; Rupniak et al, Behav Pharmacol 2001, 12:497-508; Millan et al, Neuropharmacology 2002, 42: 677-684), des états agressifs (Siegel and Schubert, Rev Neurosci 1995, 6:47-61; De Felipe et al, Nature 1998, 392: 394-397; Rupniak et al, Behav Pharmacol 2001, 12:497-508), mais aussi de l'abus de drogue (Murtra et al, Nature 2000, 405:180-183; Ripley et al, Neuropharmacology 2002, 43:1258-1268; Gadd et al, J Neurosci 2003, 23:8271-8280), des états psychotiques (Zachrisson et al, Eur Neuropsychopharmacol 2000, 10:355-363) et de l'induction des effets moteurs extrapyramidaux par les antipsychotiques (Anderson et al, J Pharmacol Exp Ther 1995, 274:928-936, Steinberg et al, J Pharmacol Exp Ther 2002, 303:1180-1188), des dysfonctions sexuelles (Priest et al, Brain Res Mol Brain Res 1995, 28:61-71; Daniels et al, Neurosci Lett 2003, 338:111-114; Kramer et al, Science 1998, 281 :1640-1644; Kramer et al, Neuropsychopharmacology 2004, 29:385-392), des perturbations des rythmes chronobiologiques tels que les rythmes circadiens (Shibata et al Brain Res 1992, 597:257-263; Challet et al, Brain Res 1998, 800:32-39; Challet et al Neuropharmacology 2001, 40:408-415; Gannon et al, Neuropharmacology, in press), de la douleur (Seguin et al, Pain 1995, 61:325-343; De Felipe et al, Nature 1998, 392:394-397; Sanger, Br J Pharmacol 2004, 141:1303-1312) et/ou l'inflammation (Seabrook et al, Eur J Pharmacol 1996, 317:129-135; Holzer, Digestion 1998, 59:269-283; Joos and Pauwels, Curr Opin Pharmacol 2001, 1:235-241; Sanger, Br J Pharmacol 2004, 141:1303-1312), de la nausée et d'autres troubles gastro-intestinaux (McAllister and Pratt Eur J Pharmacol 1998, 353:141-148; Gardner et al, Regulatory Peptides 1996, 65:45-53; Patel and Lindley, Expert Opin. Pharmacother 2003, 4:2279-2296; Sanger, Br J Pharmacol 2004, 141: 1303-1312); et en rapport avec ces éléments, des troubles de comportements et de dégénération neuronale liés à la démence et d'autres maladies du vieillissement (Raffa, Neurosci Biobehav Rev 1998, 22:789-813).

Les composés actifs à la fois sur les récepteurs $NK_1$ et sur les sites de recapture de la sérotonine (5-HT)

posséderaient des mécanismes complémentaires et synergiques pour contrôler les états impulsifs, agressifs, douloureux et surtout dépressifs. De plus, il a été montré qu'un blocage des récepteurs $NK_1$ potentialise l'influence des inhibiteurs de recapture de la 5-HT sur la transmission sérotoninergique : de ce fait, de tels composés devraient induire des effets antidépresseurs plus rapides et plus robustes (Guiard et al, J Neurochem 2004, 89:54-63; Froger et al, J Neurosci 2001, 21:8188-8197). Par ailleurs, les effets anxiolytiques rapides des antagonistes $NK_1$ seraient complémentaires des effets anxiolytiques des inhibiteurs de recapture de la 5-HT qui s'expriment après un traitement à long terme. Quant aux effets anxiogènes provoqués par la 5-HT en début de traitement (Bagdy et al, Int J Neuropsychopharmacol 2001, 4:399-408), ils devraient être prévenus par les propriétés antagonistes $NK_1$ (Ballard et al, Eur J Pharmacol 2001, 412:255-264; Rupniak et al, Neuropharmacology 2003, 44:516-523). En ce qui concerne les autres effets indésirables liés au blocage de recapture de la 5-HT, comme les effets émétisants (Goldstein and Goodnick, J Psychopharmacol 1998, 12 (Suppl B):S55-S87; Edwards and Anderson, Drugs 1999, 57:507-533; Waugh and Goa, CNS Drugs 2003, 17:343-362) et l'induction de dysfonctions sexuelles (Goldstein and Goodnick, J Psychopharmacol 1998, 12 (Suppl B):S55-S87; Montgomery et al, J Affect disord 2002, 69:119-140; Hirschfeld, J Clin Psychiatry 2003, 64 (Suppl 18):20-24), les antagonistes $NK_1$ pourraient également s'y opposer.

Par conséquent, les composés à la fois antagonistes $NK_1$ *et* inhibiteurs de recapture de la sérotonine devraient présenter des avantages thérapeutiques par rapport aux composés n'interagissant qu'avec l'une ou l'autre de ces deux cibles.

La publication Bioorganic & Medicinal Chemistry Letters 2002, Vol. 12(21), pp 3195-3198, décrit des dérivés de benzloxyphénéthylpipérazine ayant cette double activité.

Le brevet US 6 136 824 décrit des dérivés de 1-pipéridinyl-propan-2-ol et leur utilisation dans le traitement de la dépression et/ou de l'anxiété.

La publication Indian Journal of Chemistry 2000, Vol. 39B, pp 468-471 décrit des 1-amino-1,2,3,4-tétrahydronaphtalèn-2-ols et leur utilisation possible en tant qu'agents antidépresseurs et anorexigènes.

Plus spécifiquement, la présente invention concerne les composés de formule (I) :

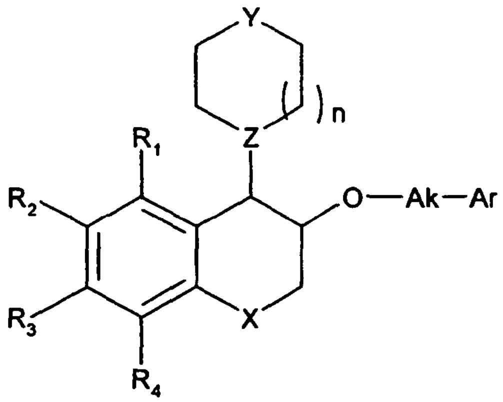

(I)

dans laquelle :

- $R_1$, $R_2$, $R_3$ et $R_4$, identiques ou différents, représentent chacun un atome ou groupement choisi parmi H, halogène, alkyle $C_1$-$C_6$ linéaire ou ramifié, alkoxy $C_1$-$C_6$ linéaire ou ramifié, phényle et cyano,

- X représente une liaison, un atome d'oxygène ou un groupement choisi parmi $-(CH_2)_m-$, $-OCH_2-$ et $NR_5-$,
  m représente 1 ou 2,
  $R_5$ représente un atome d'hydrogène ou un groupement choisi parmi alkyle
  $C_1$-$C_6$ linéaire ou ramifié, $COR_6$ et $CO_2R_6$,
  $R_6$ représente un groupement alkyle $C_1$-$C_6$ linéaire ou ramifié,

- Y représente un atome d'oxygène ou un groupement choisi parmi $NR_7$ et $CHR_8$,
  $R_7$ représente un atome d'hydrogène ou un groupement choisi parmi $COR_9$ et alkyle $C_1$-$C_6$ linéaire ou ramifié éventuellement substitué par un groupement 5-oxo-4,5-dihydro-1*H*-1,2,4-triazol-3-yle ou 2,3-dihydro-1,4-benzodioxin-2-yle,
  $R_9$ représente un groupement choisi parmi alkyle $C_1$-$C_6$ linéaire ou ramifié, aryle et hétéroaryle,
  $R_8$ représente un atome d'hydrogène ou un groupement amino éventuellement substitué par un ou deux groupements alkyle $C_1$-$C_6$ linéaire ou ramifié,

- Z représente un atome d'azote ou un groupement CH,
- n représente 1 ou 2,
- Ak représente une chaîne alkylène $C_1$-$C_6$ linéaire ou ramifiée,
- Ar représente un groupement aryle ou hétéroaryle,

leurs isomères optiques, ainsi que leurs sels d'addition à un acide pharmaceutiquement acceptable.

Par isomères optiques, on entend les énantiomères et diastéréoisomères.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, dibenzoyltartrique.

Par groupement aryle, on entend phényle, biphénylyle ou naphtyle, chacun de ces groupements étant éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano, trihalogénoalkyle $C_1$-$C_6$ linéaire ou ramifié et trihalogénoalkoxy $C_1$-$C_6$ linéaire ou ramifié.

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano et trihalogénoalkyle $C_1$-$C_6$ linéaire ou ramifié.

X représente préférentiellement une liaison, un atome d'oxygène ou un groupement-$OCH_2$- ou-$(CH_2)_m$- où m représente 1 ou 2.

Y représente préférentiellement NH.

Z représente préférentiellement un atome d'azote.

n représente préférentiellement 1.

Ar représente préférentiellement un groupement aryle.

Les composés préférés selon l'invention sont :

- la trans-1-{2-[(3,5-dibromobenzyl)oxy]-2,3-dihydro-1*H*-indén-1-yl}pipérazine, ses énantiomères, ainsi que ses sels d'addition à un acide pharmaceutiquement acceptable,
- la trans-1-{3-[(3,5-dibromobenzyl)oxy]-3,4-dihydro-2*H*-chromén-4-yl}pipérazine, ses énantiomères, ainsi que ses sels d'addition à un acide pharmaceutiquement acceptable,
- la trans-1-{6-[(3,5-dibromobenzyl)oxy]-6,7,8,9-tétrahydro-5*H*-benzo[7]annulén-5-yl}pipérazine, ses énantiomères, ainsi que ses sels d'addition à un acide pharmaceutiquement acceptable,
- la trans-1-{2-[(3,5-dibromobenzyl)oxy]-1,2,3,4-tétrahydro-naphthalén-1-yl}pipérazine, ses énantiomères, ainsi que ses sels d'addition à un acide pharmaceutiquement acceptable,
- le trans-1-{2-[(3,5-dibromobenzyl)oxy]-1,2,3,4-tétrahydro-naphthalén-1-yl}-1,4-diazépane, ses énantiomères, ainsi que ses sels d'addition à un acide pharmaceutiquement acceptable,
- la 1-{(1*S*,2*R*)-2-[(3,5-dibromobenzyl)oxy]-2,3-dihydro-1*H*-indén-1-yl}pipérazine, ses isomères optiques, ainsi que ses sels d'addition à un acide pharmaceutiquement acceptable,
- la 1-[(1S,2R)-2-[(3,5-Difluorobenzyl)-oxy]-2,3-dihydro-1*H*-indén-1-yl]-pipérazine, ses isomères optiques, ainsi que ses sels d'addition à un acide pharmaceutiquement acceptable,
- la 1-[(1S,2R)-2-[(3,5-Diméthylbenzyl)-oxy]-2,3-dihydro-1*H*-indén-1-yl]-pipérazine, ses isomères optiques, ainsi que ses sels d'addition à un acide pharmaceutiquement acceptable,
- la trans-1-{3-[(3,5-dichlorobenzyl)oxy]-3,4-dihydro-2*H*-chromén-4-yl}pipérazine, ses énantiomères, ainsi que ses sels d'addition à un acide pharmaceutiquement acceptable,
- la trans-1-{3-[3-fluoro-5-(trifluorométhyl)benzyloxy]-3,4-dihydro-2*H*-chromén-4-yl}pipérazine, ses énantiomères, ainsi que ses sels d'addition à un acide pharmaceutiquement acceptable,
- et la trans-1-{3-(3-chloro-5-fluorobenzyloxy)-3,4-dihydro-2H-chromén-4-yl}pipérazine, ses énantiomères, ainsi que ses sels d'addition à un acide pharmaceutiquement acceptable.

L'invention s'étend également au procédé de préparation des composés de formule (I) à partir du composé de formule (Va), de configuration relative trans :

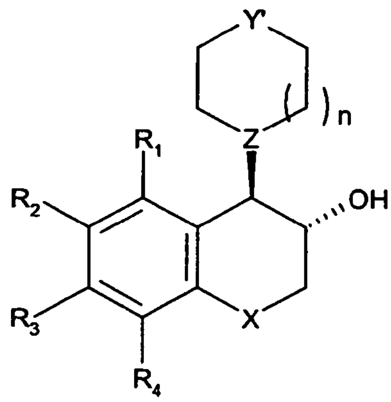

(Va)

dans laquelle $R_1$, $R_2$, $R_3$, $R_4$, X, n et Z sont tels que définis précédemment, et Y' représente un atome d'oxygène ou un groupement choisi parmi $NP_1$ et $CHR'_8$, où $R'_8$ représente un atome d'hydrogène ou un groupement $NHP_1$, et $P_1$ représente un groupement protecteur de la fonction amine,

- composé de formule (Va) qui est mis en réaction, lorsque l'on souhaite accéder aux composés de formule (I) de configuration relative trans, avec un composé de formule (VI) :

G-Ak-Ar (VI)

dans laquelle Ak et Ar sont tels que définis dans la formule (I), et G représente un groupement partant tel que, par exemple, un atome d'halogène ou un groupement p-toluènesulfonate, trifluorométhanesulfonate ou méthanesulfonate,
pour conduire au composé de formule (VIIa), de configuration relative trans :

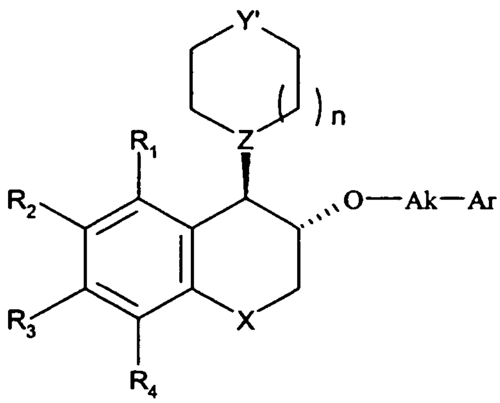

(VIIa)

dans laquelle $R_1$, $R_2$, $R_3$, $R_4$, X, n, Y', Z, Ak et Ar sont tels que définis précédemment,
que l'on déprotège, lorsque Y' comporte un groupement protecteur $P_1$ tel que défini précédemment, puis que l'on alkyle, lorsqu'on souhaite accéder à des composés pour lesquels Y représente un groupement $NR_7$ dans lequel $R_7$ est autre qu'un atome d'hydrogène,
pour conduire aux composés de formule (Ia), cas particulier des composés de formule (I) qui sont de configuration relative trans :

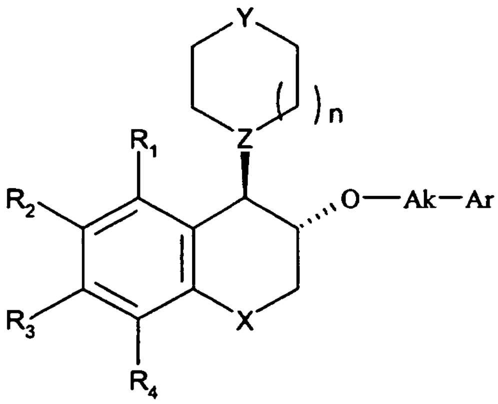

(Ia)

dans laquelle $R_1$, $R_2$, $R_3$, $R_4$, X, n, Y, Z, Ak et Ar sont tels que définis dans la formule (I),

- ou bien composé de formule (Va) qui est oxydé, lorsque l'on souhaite accéder aux composés de formule (I) de configuration relative cis, pour conduire au composé racémique de formule (VIII) :

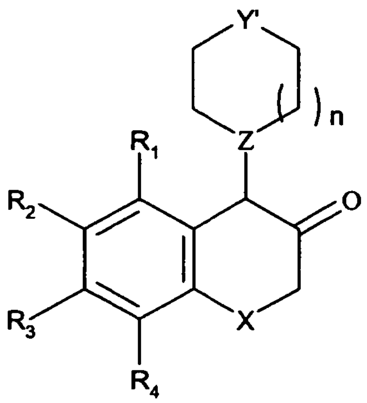

(VIII)

dans laquelle $R_1$, $R_2$, $R_3$, $R_4$, X, Z, n et Y' sont tels que définis précédemment, qui est réduit en alcool correspondant, dont les diastéréoisomères sont séparés et l'isomère de formule (Vb), de configuration relative cis, isolé :

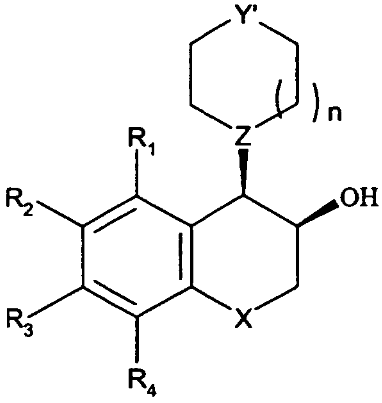

(Vb)

dans laquelle $R_1$, $R_2$, $R_3$, $R_4$, X, Y', Z et n sont tels que définis précédemment, que l'on met en réaction avec un composé de formule (VI) tel que défini précédemment, pour conduire au composé de formule (VIIb), de configuration relative cis :

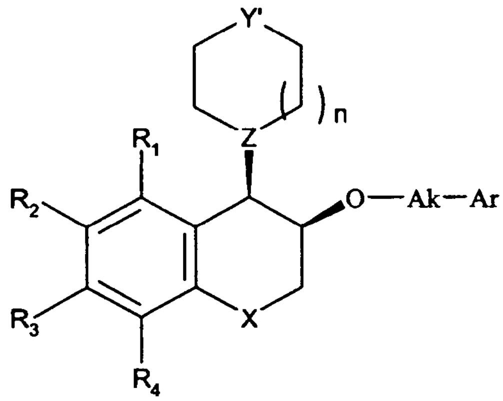

(VIIb)

dans laquelle $R_1$, $R_2$, $R_3$, $R_4$, X, n, Y', Z, Ak et Ar sont tels que définis précédemment,
que l'on déprotège, lorsque Y' comporte un groupement protecteur $P_1$ tel que défini précédemment, puis que l'on alkyle, lorsqu'on souhaite accéder à des composés pour lesquels Y représente un groupement $NR_7$ dans lequel $R_7$ est autre qu'un atome d'hydrogène,
pour conduire aux composés de formule (Ib), cas particulier des composés de formule (I) qui sont de configuration relative cis :

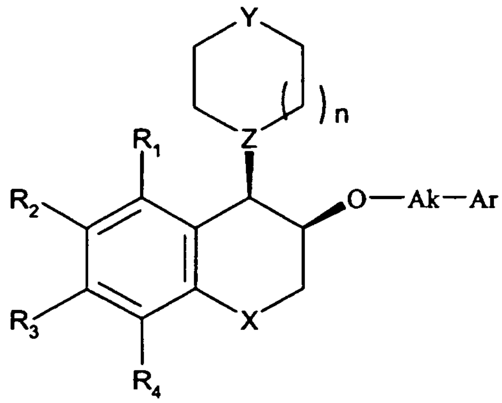

(Ib)

dans laquelle $R_1$, $R_2$, $R_3$, $R_4$, X, Y, Z, n, Ak et Ar sont tels que définis dans la formule (I),
composés de formule (Ia) et (Ib) que l'on purifie, lorsqu'on le souhaite, selon une technique classique de purification, dont on sépare, lorsqu'on le souhaite, les isomères optiques, et que l'on transforme, lorsqu'on le souhaite, en leurs sels d'addition à un acide pharmaceutiquement acceptable.

Les composés de formule (Ic), cas particulier des composés de formule (I) pour lesquels Ak représente un groupement $-CH(CH_3)-$ :

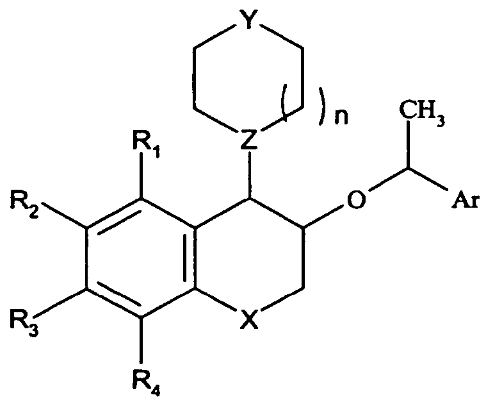

(Ic)

dans laquelle $R_1$, $R_2$, $R_3$, $R_4$, X, n, Y, Z et Ar sont tels que définis dans la formule (I), peuvent également être préparés par condensation de l'alcool de formule (Va) ou (Vb) avec un acide de formule (IX) :

$HO_2C$-Ar (IX)

dans laquelle Ar est tel que défini dans la formule (I), pour conduire à l'ester de formule (X) :

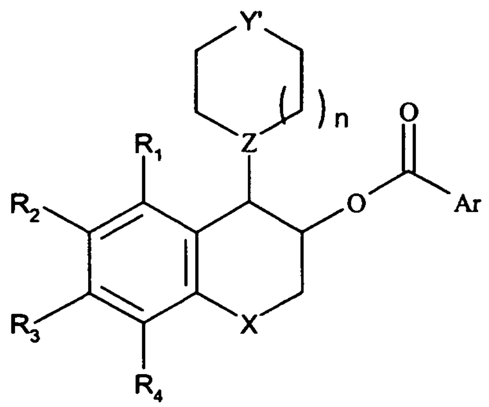

(X)

dans laquelle $R_1$, $R_2$, $R_3$, $R_4$, X, n, Y', Z et Ar sont tels que définis précédemment, que l'on met en réaction avec du bis(cyclopentadiényl)diméthyl titane, pour conduire au composé de formule (XI) :

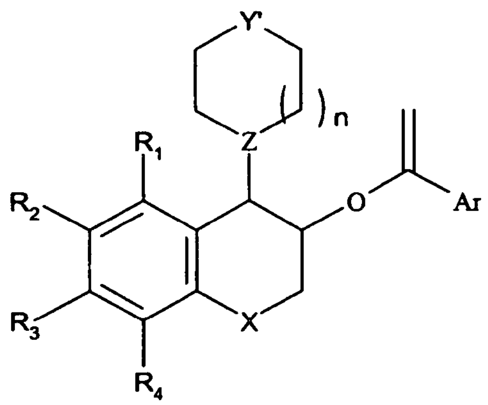

(XI)

dans laquelle $R_1$, $R_2$, $R_3$, $R_4$, X, n, Y', Z et Ar sont tels que définis précédemment, que l'on hydrogène, pour conduire au composé de formule (XII) :

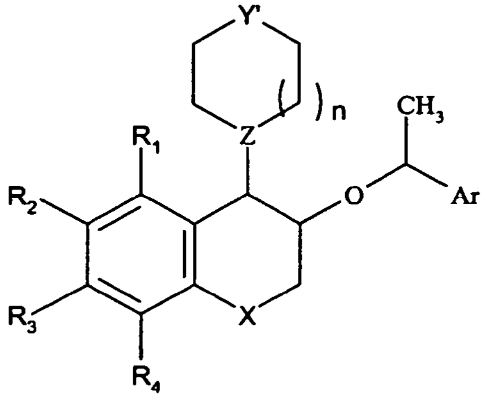

(XII)

dans laquelle $R_1$, $R_2$, $R_3$, $R_4$, X, n, Y', Z et Ar sont tels que définis précédemment,
dont on sépare les isomères, puis que l'on déprotège, lorsque Y' comporte un groupement protecteur $P_1$ tel que défini précédemment, et que l'on alkyle, lorsqu'on souhaite accéder à des composés pour lesquels Y représente un groupement $NR_7$ dans lequel $R_7$ est autre qu'un atome d'hydrogène, pour conduire aux composés de formule (Ic).

Les composés de formule ($Va_1$), cas particulier des composés de formule (Va) pour lesquels Z représente un atome d'azote, peuvent être préparés à partir du composé de formule (II) :

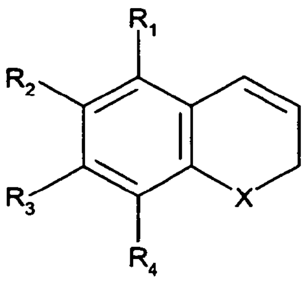

(II)

dans laquelle $R_1$, $R_2$, $R_3$, $R_4$ et X sont tels que définis dans la formule (I),
que l'on oxyde en composé de formule (III) :

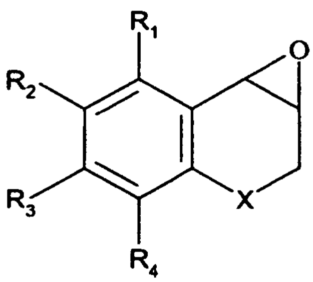

(III)

dans laquelle $R_1$, $R_2$, $R_3$, $R_4$ et X sont tels que définis précédemment,
que l'on met en réaction avec le composé de formule (IV) :

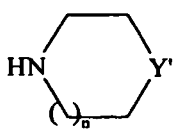

(IV)

dans laquelle n est tel que défini dans la formule (I), et Y' est tel que défini précédemment,
pour conduire au composé de formule ($Va_1$) :

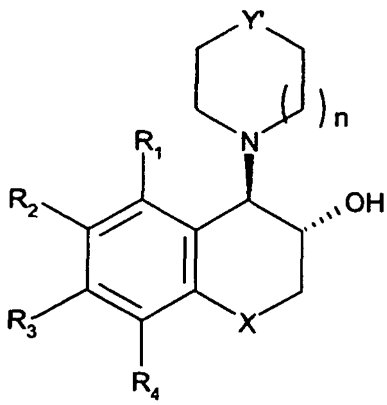

(Va$_1$)

dans laquelle $R_1$, $R_2$, $R_3$, $R_4$, X, Y' et n sont tels que définis précédemment,

Les composés de formule (Va$_2$), cas particulier des composés de formule (Va) pour lesquels Z représente un groupement CH, n représente 1, et Y' représente un groupement $NP_1$, peuvent être préparés à partir du composé de formule (XIII) :

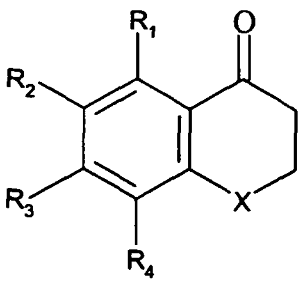

(XIII)

dans laquelle $R_1$, $R_2$, $R_3$, $R_4$ et X sont tels que définis précédemment,
que l'on met en réaction avec du 4-pyridinyllithium, pour conduire au composé de formule (XIV) :

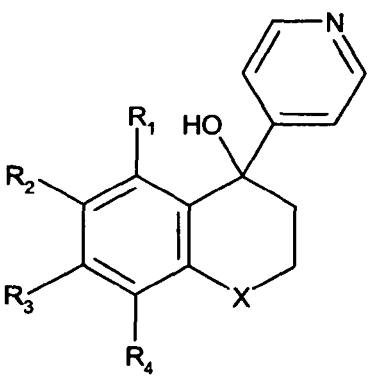

(XIV)

dans laquelle $R_1$, $R_2$, $R_3$, $R_4$ et X sont tels que définis précédemment, que l'on déshydrate, pour conduire au composé de formule (XV) :

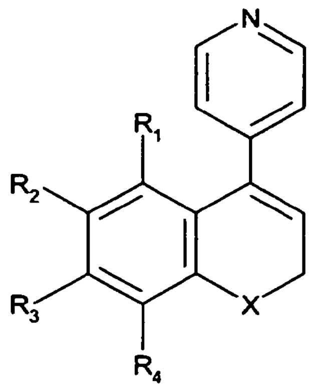

(XV)

dans laquelle $R_1$, $R_2$, $R_3$, $R_4$ et X sont tels que définis précédemment, que l'on met en réaction avec de l'Oxon®, pour conduire au composé de formule (XVI) :

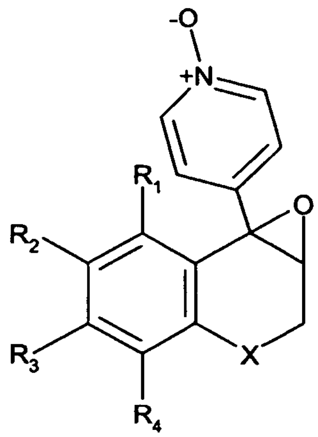

(XVI)

dans laquelle $R_1$, $R_2$, $R_3$, $R_4$ et X sont tels que définis précédemment, que l'on met en réaction avec un agent réducteur, pour conduire au composé de formule (XVII) :

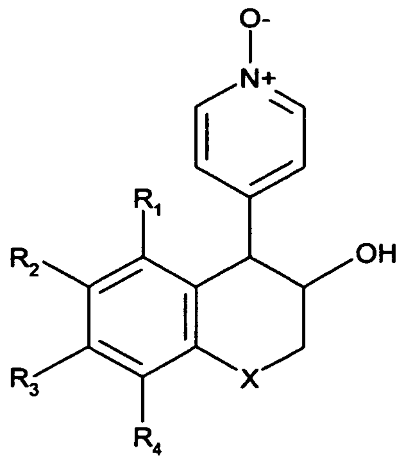

(XVII)

dans laquelle $R_1$, $R_2$, $R_3$, $R_4$ et X sont tels que définis précédemment, que l'on soumet à une réaction d'hydrogénation catalytique, pour conduire au composé de formule (XVIII) :

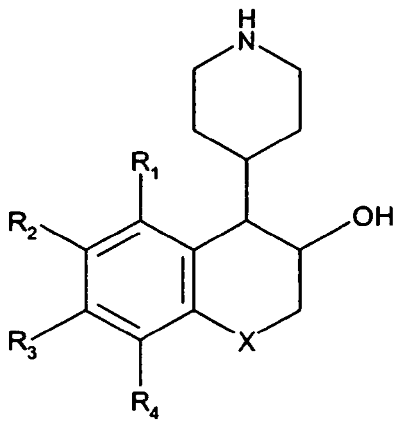

(XVIII)

dans laquelle $R_1$, $R_2$, $R_3$, $R_4$ et X sont tels que définis précédemment,
dont on sépare les isomères cis et trans puis protège la fonction amine de l'isomère trans, pour conduire au composé de formule ($Va_2$), de configuration relative trans :

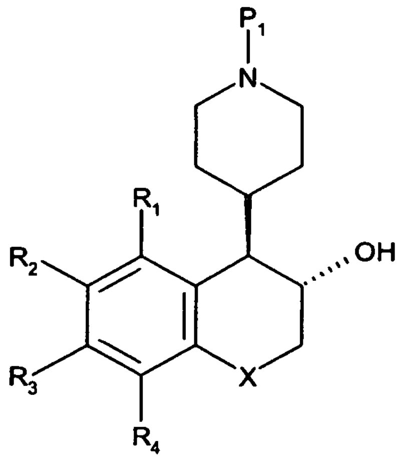

($Va_2$)

dans laquelle $R_1$, $R_2$, $R_3$, $R_4$ et X sont tels que définis précédemment.

Les composés de la présente invention sont des inhibiteurs de la recapture de la sérotonine, et la plupart d'entre eux sont également des antagonistes $NK_1$. Ils sont utiles, en tant que médicament, pour le traitement des états dépressifs, des états anxieux, des troubles impulsifs, des comportements agressifs, de l'abus de drogue, de l'obésité et des troubles de l'appétit, de la douleur et l'inflammation, des démences, des états psychotiques, des perturbations des rythmes chronobiologiques, de la nausée et des troubles gastro-intestinaux.

La présente invention a également pour objet les compositions pharmaceutiques contenant comme principe actif un composé de formule (I), ou son sel d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse).

Les points de fusion ont été déterminés, soit au banc de Kofler (BK), soit à la platine chauffante sous microscope (MK).

**PREPARATION A : 6-Bromo-1,2-dihydronaphthalène**

*Stade A : 7-Bromo-1,2,3,4-tétrahydronaphthalèn-1-ol*

A 9,5g de 7-bromo-3,4-dihydronaphthalèn-1(2*H*)-one (42 mmoles), préparée selon la méthode décrite dans Synth. Comm. 1994, 2777, en solution dans 100 ml d'éthanol, sont ajoutés à 0°C et en deux fois 0,8 g de borohydrure de sodium (21 mmoles). On laisse ensuite remonter le milieu réactionnel à température ambiante en 30 minutes, puis évapore l'éthanol. Le résidu est repris par 100 ml de toluène et 100 ml d'eau. Après décantation, la phase aqueuse est extraite par 50 ml de toluène. Les phases toluéniques sont réunies, lavées par une solution saturée de chlorure de sodium aqueux, puis évaporées pour conduire au 7-bromo-1,2,3,4-tétrahydronaphthalèn-1-ol sous forme d'huile.

*Stade B : 6-Bromo-1,2-dihydronaphthalène*

On porte à 100°C une solution de 8,6 g du composé obtenu au stade précédent (37,9 mmoles) dans 200 ml de toluène. A cette température, on ajoute goutte à goutte, en une heure, une solution de 0,3 g d'acide paratoluènesulfonique (1,2 mmole) en solution dans 400 ml de toluène. Le mélange réactionnel est ensuite refroidi à 25°C, puis hydrolysé avec 100 ml d'eau. La phase organique est extraite puis séchée, filtrée et évaporée, pour conduire au 6-bromo-1,2-dihydronaphthalène sous forme d'huile.

**PREPARATION B : 7-Bromo-2,3-dihydro-1-benzoxépine**

*Stade A : 4-[5-Bromo-2-(but-3-én-1-yloxy)phényl]-4-hydroxybutan-2-one*

A 300 ml d'acétone sont ajoutés 10 g de 5-bromo-2-hydroxybenzaldehyde (49,7 mmoles), 13,7 g de carbonate de potassium (99,5 mmoles) et 10,1 ml de 4-bromo-but-1-ène (99,5 mmoles), puis le mélange réactionnel est porté à reflux pendant 36 heures avant d'être refroidi, filtré et évaporé à sec, pour conduire à la 4-[5-bromo-2-(but-3-én-1-yloxy)phényl]-4-hydroxybutan-2-one sous forme d'huile.

*Stade B : 4-Bromo-1-(but-3-én-1-yloxy)-2-vinylbenzène*

A 90 ml de tétrahydrofurane anhydre, sont ajoutés 1,6 g d'hydrure de sodium à 60% dans l'huile (39,8 mmoles), puis, à 0°C et en une fois, 10,67g de bromure de méthyl(triphényl)phosphonium (29,9 mmoles).

On laisse remonter à température ambiante et on agite 30 minutes à 25°C. Puis on verse goutte à goutte sur ce milieu réactionnel, à température ambiante, une solution de 8g du composé obtenu au stade précédent (24,9 mmoles) dans 30 ml de tétrahydrofurane anhydre. On observe une exothermie de 25°C à 35°C en 45 minutes. On laisse encore agiter 2 heures à température ambiante, puis on filtre le milieu réactionnel, et le filtrat est versé sur un mélange de 100 ml d'acétate d'éthyle, 200 ml d'une solution aqueuse saturée de chlorure de sodium et 50 g de glace. Après extraction par l'acétate d'éthyle, les phases organiques réunies sont lavées à l'eau, séchées, filtrées et évaporées à sec. Le résidu obtenu est purifié par filtration sur 100g de silice (éluant : toluène 100%), pour conduire au 4-bromo-1-(but-3-én-1-yloxy)-2-vinylbenzène sous forme d'huile.

*Stade C : 7-Bromo-2,3-dihydro-1-benzoxépine*

5g du composé obtenu au stade précédent (19,8 mmoles) sont solubilisés dans 500 ml de toluène, puis la solution est dégazée à l'azote pendant 30 minutes. On ajoute 335 mg de [1,3-bis-(2,4,6-triméthylphényl)-2-imidazolidinylidène)dichloro(phénylméthylène)-(tricyclohexyl-phosphine)ruthénium], ou catalyseur de Grubb's II (0,39mmoles). Le milieu réactionnel est ensuite chauffé à 50°C pendant 30 minutes, puis le toluène est évaporé et le résidu obtenu est purifié sur une colonne de 70g de silice (éluant : cyclohexane-toluène : 95-5), pour conduire à la 7-bromo-2,3-dihydro-1-benzoxépine.

**EXEMPLE 1 : Trans-1-{2-[(3,5-dibromobenzyl)oxy]-2,3-dihydro-1H indén-1-yl}pipérazine, dichlorhydrate**

*Stade A : Trans-4-(2-hydroxy-2,3-dihydro-1H-indén-1-yl)pipérazine-1-carboxylate de tert-butyle*

On solubilise dans 30 ml d'acétonitrile 11,6 g de pipérazine-1-carboxylate de *tert*-butyle (62 mmoles) et 8,2 g d'oxyde d'indène (62 mmoles). Le milieu réactionnel est ensuite chauffé à 60°C pendant une nuit, puis évaporé à sec. Le résidu obtenu est purifié par flash-chromatographie sur 1 kg de silice (éluant : dichlorométhane/éthanol 95/5), pour conduire au trans-4-(2-hydroxy-2,3-dihydro-1*H*-indén-1-yl)pipérazine-1-carboxylate de *tert*-butyle sous forme de meringue blanche.

*Stade B : Trans-4-{2-[(3,5-dibromobenzyl)oxy]-2,3-dihydro-1H-indén-1-yl}pipérazine-1-carboxylate de tert-butyle*

A 3 g du composé obtenu au stade précédent (9,42 mmoles) dans 30 ml de diméthylformamide anhydre sont ajoutés 452 mg d'hydrure de sodium en suspension à 60% dans l'huile (11,3 mmoles, 1,2 équivalents). Après 30 minutes d'agitation à température ambiante sont ajoutés 3,1 g de bromure de 3,5-dibromobenzyle (9,42 mmoles). On observe une légère exothermie. Le milieu réactionnel est ensuite agité pendant une nuit à température ambiante, puis la diméthylformamide est évaporée. Le résidu obtenu est repris au dichlorométhane. Après lavage à l'eau, séchage, filtration et évaporation, on obtient 6g d'un résidu qui est purifié par flash-chromatographie sur 500g de silice (éluant : dichlorométhane / acétate d'éthyle 90/10), pour conduire au trans-4-{2-[(3,5-dibromobenzyl)oxy]-2,3-dihydro-1*H*-indén-1-yl} pipérazine-1-carboxylate de *tert*-butyle sous forme de meringue.

*Stade C : Trans 1-{2-[(3,5-dibromobenzyl)oxy]-2,3-dihydro-1H-indén-1-yl}pipérazine, dichlorhydrate*

On solubilise 3,5 g du composé obtenu au stade précédent (6,18 mmoles) dans 250 ml d'acétate d'éthyle, puis on fait buller au travers de la solution de l'acide chlorhydrique gazeux. On laisse la température monter jusqu'à 45°C, puis on laisse agiter 2 heures à température ambiante. Le milieu réactionnel est alors concentré aux deux tiers, puis 50 ml d'éther sont ajoutés. Le précipité obtenu est filtré, puis séché, pour conduire au dichlorhydrate de la trans-1-{2-[(3,5-dibromobenzyl)oxy]-2,3-dihydro-1*H*-indén-1-yl}pipérazine sous la forme d'un solide beige.
*Point de fusion* (MK) : 154-167°C.

**EXEMPLE 2 : Isomère (-) de la trans-1-{2-[(3,5-dibromobenzyl)oxy]-2,3-dihydro-1*H*-indén-1-yl}pipérazine, dichlorhydrate**

Après retour base, le composé racémique de l'Exemple 1 est séparé par chromatographie HPLC préparative chirale (éluant : isopropanol/ acétonitrile/ diéthylamine 100/900/1) sur phase Chiralpak AD. Le premier des isomères ainsi séparé est salifié par l'acide chlorhydrique, pour conduire au dichlorhydrate de l'isomère (-) de la trans-1-{2-[(3,5-dibromobenzyl)oxy]-2,3-dihydro-1*H*-indén-1-yl}pipérazine.
*Point de fusion* (MK) : 117-125°C.
*Pouvoir rotatoire :* $[\alpha]_D$= - 27,86 (c=1%, MeOH, 20°C, 589nm).

**EXEMPLE 3 : Isomère (+) de la trans-1-{2-[(3,5-dibromobenzyl)oxy]-2,3-dihydro-1H-indén-1-yl}pipérazine, dichlorhydrate**

Le deuxième des isomères séparés à l'Exemple 2 est salifié par l'acide chlorhydrique, pour conduire au dichlorhydrate de l'isomère (+) de la trans-1-{2-[(3,5-dibromobenzyl)oxy]-2,3-dihydro-1*H*-indén-1-yl}pipérazine.
*Point de fusion* (MK) : 115-121 °C
*Pouvoir rotatoire :* $[\alpha]_D$ = + 27,29 (c=1%, MeOH, 20°C, 589nm)

**EXEMPLE 4 : Trans-1-{3-[(3,5-dibromobenzyl)oxy]-3,4-dihydro-2*H*-chromén-4-yl}pipérazine, dichlorhydrate**

*Stade A : Trans-3-bromochroman-4-ol*

A 15 g de 2*H*-chromène (0,113 mole) en solution dans 330 ml d'un mélange tétrahydrofurane / eau 50/50 sont ajoutés 22,1g de N-bromosuccinimide (0,124 mole, 1,1 équivalent), puis le mélange est agité pendant une heure à température ambiante. Le milieu réactionnel est ensuite dilué à l'eau, puis extrait 2 fois à l'acétate d'éthyle. Les phases organiques réunies sont lavées à l'eau, séchées, filtrées et évaporées à sec, pour conduire au trans-3-bromochroman-4-ol sous forme d'un solide jaune pâle.
*Point de fusion* (BK) : 96-98°C.

*Stade B : 1a,7b-Dihydro-2H-oxiréno[c]chromène*

A 10g du composé obtenu au stade précédent (43,6 mmoles) en solution dans 170 ml de tétrahydrofurane et 85 ml d'eau sont ajoutés à température ambiante 4,4g de potasse en pastille (78,5 mmoles). Après agitation pendant 2 heures à 25°C, le milieu réactionnel est dilué à l'eau et extrait 2 fois à l'éther. Les phases organiques sont réunies et lavées à l'eau, séchées, filtrées et évaporées à sec pour conduire au 1a, 7b-dihydro-2*H*-oxiréno[c]chromène sous forme d'huile jaune pâle.

*Stade C : Trans-4-(3-hydroxy-3,4-dihydro-2H-chromén-4-yl)pipérazine-1-carboxylate de tert-butyle*

4,9 g du composé obtenu au stade précédent (33 mmoles) sont traités par 6,1 g de pipérazine-1-carboxylate de *tert*-butyle (33 mmoles) selon la méthode décrite dans le stade A de l'Exemple 1. L'huile obtenue est purifiée par flash-chromatographie sur 500g de silice (éluant : dichlorométhane / éthanol 95/5), pour conduire au produit attendu sous forme d'huile.

*Stade D : Trans-4-{3-[(3,5-dibromobenzyl)oxy]-3,4-dihydro-2H-chromén-4-yl} pipérazine-1- carboxylate de tert-butyle*

3 g du composé obtenu au stade précédent (8,9 mmoles) sont traités selon la méthode décrite dans le stade B de l'Exemple 1. L'huile obtenue est purifiée par flash-chromatographie sur 300g de silice pour conduire au produit attendu sous forme de meringue blanche.

*Stade E : Trans-1-{3-[(3,5-dibromobenryl)oxy]-3,4-dihydro-2H chromén-4-yl}pipérazine, dichlorhydrate*

2,9 g du composé obtenu au stade précédent (4,97 mmoles) sont traités selon la méthode décrite dans le stade C de l'Exemple 1. Le produit obtenu est cristallisé dans l'éther, filtré et séché pour conduire au produit attendu sous forme de cristaux blanc.
*Point de fusion* (MK) : 130-135°C.

**EXEMPLE 5 : Isomère (-) de la trans-1-{3-[(3,5-dibromobenzyl)oxy]-3,4-dihydro-2*H*-chromén-4-yl}pipérazine, dichlorhydrate**

Après retour base, le composé racémique de l'Exemple 4 est séparé par chromatographie HPLC préparative chirale (éluant : éthanol/ diéthylamine 1000/1) sur phase Chiralpak AD. Le premier des isomères ainsi séparé est salifié par l'acide chlorhydrique, pour conduire au dichlorhydrate de l'isomère (-) de la trans-1-{3-[(3,5-dibromobenzyl)oxy]-3,4-dihydro-2*H*-chromén-4-yl}pipérazine.
*Point de fusion* (MK) : 128-132°C.
*Pouvoir rotatoire :* $[\alpha]_D$ = - 40,36 (c=1%, MeOH, 20°C, 589nm).

**EXEMPLE 6 : Isomère (+) de la trans-1-{3-[(3,5-dibromobenzyl)oxy]-3,4-dihydro-2*H*-chromén-4-yl)pipérazine, dichlorhydrate**

Le deuxième des isomères séparés à l'Exemple 5 est salifié par l'acide chlorhydrique, pour conduire au dichlorhydrate de l'isomère (+) de la trans-1-{3-[(3,5-dibromobenzyl)oxy]-3,4-dihydro-2*H*-chromén-4-yl}pipérazine.
*Point de fusion* (MK) : 126-130°C.
*Pouvoir rotatoire* : $[\alpha]_D$ = + 40,92 (c=1%, MeOH, 20°C, 589nm).

**EXEMPLE 7 : Trans-1-{6-[(3,5-dibromobenzyl)oxy]-6,7,8,9-tétrahydro-5*H*-benzo[7]annulén-5-yilpipérazine, dichlorhydrate**

*Stade A : Trans-4-(6-hydroxy-6,7,8,9-tétrahydro-5H-benzo[7]annulén-5-yl)pipérazine-1-carboxylate de tert-butyle*

Le produit attendu est obtenu selon le procédé décrit aux stades A, B et C de l'Exemple 4, en remplaçant le 2*H*-chromène par le 6,7-dihydro-5*H*-benzo[7]annulène.

*Stade B : Trans-1-{6-[(3,5-dibromobenzyl)oxy]-6,7,8,9-tétrahydro-5H-benzo[7]annulén-5-yl}pipérazine, dichlorhydrate*

Le produit attendu est obtenu selon le procédé décrit aux stades B et C de l'Exemple 1, à partir du composé obtenu au stade précédent.

*Point de fusion* (MK) : 126-131°C.

**EXEMPLE 8 : Trans-1-{4-[(3,5-dibromobenzyl)oxy]-2,3,4,5-tétrahydro-1-benzoxépin-5-yl}pipérazine, dichlorhydrate**

*Stade A : 1a,2,3,8b-Tétrahydrooxiréno[d][1]benzoxépine*

A 0,5g de 2,3-dihydro-1-benzoxépine (3,42 mmoles), préparée selon la méthode décrite dans J. Org. Chem., 1969, 34 (1), 207, en solution dans 30 ml d'un mélange acétate d'éthyle/ eau 50/50, sont ajoutés 1,44g d'hydrogénocarbonate de sodium (17,1 mmoles), puis, en 1 heure, une solution de 2,1g d'Oxone® (3,42 mmoles) dans 15 ml d'eau. On laisse agiter 1 heure supplémentaire après la fin d'addition, puis on décante la phase organique. La phase aqueuse est de nouveau extraite avec 10 ml d'acétate d'éthyle, les phases organiques réunies sont lavées à l'eau, séchées, filtrées et évaporées à sec, pour conduire au produit attendu sous forme d'huile.

*Stade B : Trans-4-(4-hydroxy-2,3,4,5-tétrahydro-1-benzoxépin-5-yl)pipérazine-1-carboxylate de tert-butyle*

Le produit attendu est obtenu selon le procédé décrit au stade A de l'Exemple 1, à partir du composé obtenu au stade précédent.

***Stade C*** *: Trans-1-{4-[(3,5-dibromobenzyl)oxy]-2,3,4,5-tétrahydro-1-benzoxépin-5-yl}pipérazine, dichlorhydrate*

Le produit attendu est obtenu selon le procédé décrit aux stades B et C de l'Exemple 1, à partir du composé obtenu au stade précédent.
*Point de fusion* (MK) : 132-139°C.

**EXEMPLE 9 : Trans-1-{3-[(3,5-dibromobenzyl)oxy]-3,4-dihydro-2*H*-chromén-4-yl}pipéridin-4-amine, dichlorhydrate**

*Stade A : Trans-[1-(3-hydroxy-3,4-dihydro-2H-chromén-4-yl)pipéridin-4-yl] carbamate de tert-butyle*

A 3g du composé obtenu au stade B de l'Exemple 4 en solution dans 45 ml d'acétonitrile sont ajoutés 4g de pipéridin-4-ylcarbamate de *tert-butyle* (20,2 mmoles). Le mélange est ensuite chauffé à reflux pendant 12 heures, puis évaporé à sec. On obtient une huile jaune qui est purifiée par flash-chromatographie sur silice (éluant : dichlorométhane/ éthanol 95/5), pour conduire au produit attendu sous forme de meringue jaune pâle.

*Stade B: Trans-1-{3-[(3,5-dibromobenzyl)oxy]-3,4-dihydro-2H-chromén-4-yl}-4-pipéridinylcarbamate de tert-butyle*

Le produit attendu est obtenu selon le procédé décrit au stade B de l'Exemple 1, à partir du composé obtenu au stade précédent.

*Stade C: Trans-1-{3-[(3,5-dibromobenzyl)oxy]-3,4-dihydro-2H-chromén-4-yl)pipéridin-4-amine, dichlorhydrate*

Le produit attendu est obtenu selon le procédé décrit au stade C de l'Exemple 1, à partir du composé obtenu au stade précédent.
*Point de fusion* (MK) : 160-165°C.

**EXEMPLE 10 : Trans-4-{2-[(3,5-dibromobenzyl)oxy]-1,2,3,4-tétrahydro-naphthalén-1-yl}morpholine, chlorhydrate**

*Stade A : 1a,2,3,7b-Tétrahydronaphtho[1,2-b]oxirène*

36,5g de 1,2-dihydronaphthalène (280,3 mmoles) sont traités par l'Oxone® selon la méthode décrite dans le stade A de l'Exemple 8, pour conduire au produit attendu sous forme d'huile.

*Stade B : Trans 1-morpholin-4-yl-1,2,3,4-tétrahydronaphthalén-2-ol*

A 3,95g du composé obtenu au stade précédent (27,02 mmoles) en solution dans 65 ml d'acétonitrile sont ajoutés 2,35ml de morpholine (27,02 mmoles), puis 185mg de $ZnCl_2$ (1,35 mmole). Après 12h de chauffage à reflux, le

milieu est évaporé à sec et le résidu obtenu purifié par flash-chromatographie sur silice (dichlorométhane/ éthanol 95/5) pour conduire au produit attendu sous forme d'un solide marron clair.
*Point de fusion* (BK) : 88-90°C.

*Stade C : Trans-4-{2-[(3,5-dibromobenzyl)oxy]-1,2,3,4-tétrahydro-naphthalén-1-yl}morpholine*

3 g du composé obtenu au stade précédent (12,85 mmoles) sont traités selon la méthode décrite dans le stade B de l'Exemple 1. On obtient une huile qui est purifiée par flash-chromatographie sur silice (éluant : dichlorométhane) pour conduire au produit attendu sous forme d'huile.

*Stade D : Trans-4-{2-[(3,5-dibromobenzyl)oxy]-1,2,3,4-tétrahydro-naphthalén-1-yl}morpholine, chlorhydrate*

A 1,9 g du composé obtenu au stade précédent (3,95 mmoles) en solution dans 40 ml d'acétate d'éthyle sont ajoutés 2,96 ml d'une solution 2 M d'éther chlorhydrique (5,92 mmoles). Après filtration et séchage des cristaux, on obtient le produit attendu.
*Point de fusion* : (MK) = 175-180°C.

**EXEMPLE 11 : Trans-1-{2-[(3,5-dibromobenzyl)oxy]-5-méthoxy-2,3-dihydro-1*H*-indén-1-yl} pipérazine**

*Stade A : 4-Méthoxy-6,6a-dihydro-1aH-indéno[1,2-b]oxirène*

Le produit attendu est obtenu selon le procédé décrit aux stades A et B de l'Exemple 4, à partir de 6-méthoxy-1*H*-indène.

*Stade B : Trans-4-{2-[(3,5-dibromobenzyl)oxy]-5-méthoxy-2,3-dihydro-1H-indén-1-yi}pipérazine-1-carboxylate de tert-butyle*

Le produit attendu est obtenu selon le procédé décrit aux stades A et B de l'Exemple 1, à partir du composé obtenu au stade précédent.

*Stade C : Trans-1-{2-[(3,5-dibromobenzyl)oxy]-5-méthoxy-2,3-dihydro-1H-indén-1yl} pipérazine*

1,3 g du composé obtenu au stade précédent (2,17 mmoles) sont traités selon la méthode décrite dans le stade C de l'Exemple 1. Après évaporation à sec, le produit obtenu est repris à l'eau, la phase aqueuse est amenée à pH 8 par ajout de soude (1 N) et extraite à l'éther. La phase organique est lavée à l'eau, séchée, filtrée et évaporée à sec. Le résidu obtenu est cristallisé dans l'éther isopropylique. Les cristaux sont filtrés et séchés pour conduire au produit attendu.
*Point de fusion* (MK) : 101-104°C.

**EXEMPLE 12 : Trans-1-{2-[(3,5-dibromobenzyl)oxy]-7-méthoxy-1,2,3,4-tétrahydronaphthalén-1-yl}pipérazine, dichlorhydrate**

*Stade A : 2-Bromo-7-méthoxy-1,2,3,4-tétrahydronaphthalén-1-ol*

20 g de 6-méthoxy-1,2-dihydronaphthalène (126 mmoles) sont traités selon la méthode décrite dans le stade A de l'Exemple 4, pour conduire au produit attendu.

*Stade B : Trans-4-(2-hydroxy-7-méthoxy-1,2,3,4-tétrahydronaphthalén-1-yl)pipérazine-1-carboxylate de tert-butyle*

A 2 g du composé obtenu au stade précédent (7,78 mmoles) en solution dans 30 ml de tétrahydrofurane sont ajoutés 1,24 g d'hydrure de sodium en suspension à 60% dans l'huile (31,1 mmoles) et on agite 4 heures à température ambiante. On filtre le milieu réactionnel. Le filtrat qui contient le 6-méthoxy-1a,2,3,7b-tétrahydronaphtho[1,2-*b*]oxirène en solution est alors mis en réaction avec 1,5 g de pipérazine-1-carboxylate de *tert*-butyle (7,78 mmoles) et 5 ml de diméthylformamide. On distille le tétrahydrofurane du milieu réactionnel puis on porte à 110°C pendant 24 heures. On refroidit, verse sur de l'eau et extrait au dichlorométhane. La phase organique est séchée, puis filtrée et évaporée à sec. Le résidu obtenu est purifié par flash-chromatographie sur silice (éluant : dichlorométhane/éthanol : 98/2), pour conduire au produit attendu.

*Stade C : Trans-1-{2-[(3,5-dibromobenzyl)oxy]-7-méthoxy-1,2,3,4-tétrahydronaphthalén-1-yl}pipérazine, dichlorhydrate*

Le produit attendu est obtenu selon le procédé décrit aux stades B et C de l'Exemple 1, à partir du composé obtenu au stade précédent.
Point de fusion (MK) : 175-182°C.

**EXEMPLE 13 : Trans-1-{2-[(3,5-dibromobenzyl)oxy]-6-méthoxy-1,2,3,4-tétrahydronaphthalén-1-yl}pipérazine, dichlorhydrate**

*Stade A : 2-Bromo-6-méthoxy-1,2,3,4-tétrahydronaphthalén-1-ol*

10,8 g de 7-méthoxy-1,2-dihydronaphthalene (67,4 mmoles) sont traités selon la méthode décrite dans le stade A de l'Exemple 4, pour conduire au produit attendu.

*Stade B : Trans-4-(2-hydroxy-6-méthoxy-1,2,3,4-tétrahydronaphthalén-1-yl)pipérazine-1-carboxylate de tert-butyle*

A 9,7 g du composé obtenu au stade précédent (37,8 mmoles) en solution dans 150 ml de tétrahydrofurane sont ajoutés 3 g d'hydrure de sodium en suspension à 60% dans l'huile (75,6 mmoles, 2 équivalents), puis le mélange réactionnel est agité pendant 24 heures à température ambiante. Après filtration, le filtrat, qui contient le 5-méthoxy-1a, 2,3,7b-tétrahydronaphtho[1,2-*b*]oxirène en solution, est mis en réaction avec 8,4 g de pipérazine-1-carboxylate de *tert*-butyle (45,3 mmoles) dans 20 ml de diméthylformamide. Le tétrahydrofurane est ensuite distillé, puis le milieu réactionnel est porté à 110°C pendant 1 heure. On refroidit alors le mélange réactionnel avant de le verser sur de l'eau et de l'extraire au dichlorométhane. La phase organique est ensuite séchée, filtrée et évaporée à sec pour conduire à un résidu qui est purifié par flash-chromatographie sur silice (éluant : dichlorométhane/ éthanol : 98/2), pour conduire au produit attendu.

*Stade C: Trans-1-{2-[(3,5-dibromobenzyl)oxy]-6-méthoxy-1,2,3,4-tétrahydronaphthalén-1-yl}pipérazine, dichlorhydrate*

Le produit attendu est obtenu selon le procédé décrit aux stades B et C de l'Exemple 1, à partir du composé obtenu au stade précédent.
Point de fusion (MK) : 137-150°C.

**EXEMPLE 14 : Trans-1-{2-[(3,5-dibromobenzyl)oxy]-5-phényl-2,3-dihydro-1*H*-indén-1-yl}pipérazine, dichlorhydrate**

Le produit attendu est obtenu selon le procédé de l'Exemple 4, à partir de 6-phényl-1*H*-indène.
Point de fusion (MK) : 145-153°C.

**EXEMPLE 15 : Trans-1-{2-[(3,5-dibromobenzyl)oxy]-1,2,3,4-tétrahydro-naphthalén-1-yl}pipérazine, dichlorhydrate**

*Stade A : Trans-2-bromo-1,2,3,4-tétrahydronaphthalén-1-ol*

Sur 13,0g (100 mmoles) de dihydronaphtalène en solution dans un mélange de 100 ml d'eau et 400 ml de tétrahydrofurane, à température ambiante, on ajoute d'un trait 19,6g (110 mmoles) de N-bromosuccinimide et agite pendant 3h. On ajoute alors 200 ml d'eau glacée et 200 ml d'éther, agite et sépare les phases. La phase aqueuse est extraite par 200 ml d'éther, puis les phases organiques jointes sont lavées par 200 ml d'une solution aqueuse saturée de chlorure de sodium et séchées puis évaporées, pour conduire au composé attendu.

***Stade B** : 1a,2,3,7b-Tétrahydronaphtho[1,2-b]oxirène*

A 27,5g du composé obtenu au stade précédent en solution dans 180 ml de tétrahydrofurane, est ajoutée goutte à goutte en 5 minutes, à température ambiante, une solution de 10g (180 mmoles) de potasse dans 10 ml d'eau. Après 5 jours d'agitation à température ambiante, on ajoute 200 ml d'eau et extrait par de l'éther éthylique. Les phases organiques jointes sont lavées puis séchées. Après évaporation, le produit obtenu est distillé sous vide (0,1 mm Hg environ) au Kugelrohr. Le produit attendu distille vers 80°C.

*Stade C : 4-[Trans-2-hydroxy-1,2,3,4-tétrahydronaphthalén-1-yl]pipérazine-1-carboxylate de tert-butyle*

A température ambiante, on mélange 4,4g (30 mmoles) du composé obtenu au stade précédent et 5,6g (30 mmoles) de N-tert-butyloxycarbonyl pipérazine dans 60 ml d'acétonitrile, puis on porte à reflux pendant 40h. Après évaporation et flash chromatographie sur 500g de silice (éluant : dichlorométhane/éthanol 98/2), on recueille le composé attendu.

*Stade D : 4-{trans-2-[(3,5-dibromobenzyl)oxy]-1,2,3,4-tétrahydronaphthalén-1-yl}pipérazine-1-carboxylate de tert-butyle*

A température ambiante, sur 0,44g (11 mmoles) d'hydrure de sodium en suspension dans 20 ml de tétrahydrofurane, on ajoute en quelques minutes 3,3g (10 mmol) du composé obtenu au stade précédent en solution dans 20 ml de tétrahydrofurane. On porte 2h à 50°C, puis ajoute d'un trait 3,3g (10 mmoles) de bromure de 3,5-dibromobenzyle, puis 3,7g (10 mmoles) d'iodure de tétra-n-butyl ammonium, et on chauffe pendant la nuit à 70°C. On verse alors dans 100 ml d'eau et extrait 2 fois par 100 ml d'éther éthylique. Les phases organiques jointes sont lavées et séchées. Après évaporation et flash chromatographie sur 300g de silice (éluant : dichlorométhane 100%, puis dichlorométhane/acétate d'éthyle 90/10), on recueille le composé attendu.

*Stade E : Trans-1-{2-[(3,5-dibromobenzyl)oxy]-1,2,3,4-tétrahydro-naphthalén-1-yl}pipérazine, dichlorhydrate*

Sur 5,8g (10 mmoles) du composé précédent en solution dans 400 ml d'acétate d'éthyle, à température ambiante, on fait buller doucement pendant quelques minutes de l'acide chlorhydrique gazeux. On maintient pendant 3h sous agitation à température ambiante, puis on concentre de moitié (apparition de précipité) et ajoute 100 ml d'éther, puis maintient la nuit sous agitation à température ambiante. Après filtration, rinçage et séchage du précipité, on recueille le composé attendu sous forme de racémique.
*Point de fusion* (M.K.) : 110-125°C.

**EXEMPLE 16 : Isomère (-) de la trans-1-{2-[(3,5-dibromobenzyl)oxy]-1,2,3,4-tétrahydro-naphthalén-1-yl}pipérazine, dichlorhydrate**

Après retour base, le composé racémique obtenu à l'Exemple 15 est séparé par chromatographie HPLC chirale préparative sur colonne CHIRALPAK AD (éluant : acétonitrile/isopropanol/diéthylamine 900/100/1). Le premier des isomères ainsi séparés est salifié par chlorhydratation par l'éthanol chlorhydrique, puis précipitation dans l'éther. Après filtration, rinçage et séchage, on obtient l'énantiomère (-) avec un excès énantiomérique supérieur à 98%.
*Point de fusion* (M.K.) : 113-126°C.
*Pouvoir rotatoire :* [$\alpha$]= - 14,5 (c=1%, MeOH, 20°C, $\lambda$=589nm)

**EXEMPLE 17 : Isomère (+) de la trans-1-{2-[(3,5-dibromobenzyl)oxy]-1,2,3,4-tétrahydro-naphthalén-1-yl}pipérazine, dichlorhydrate**

Le deuxième des isomères séparés à l'Exemple 16 est transformé en chlorhydrate, pour conduire au produit attendu.
*Point de fusion* (M.K.) : 118-126°C.
*Pouvoir rotatoire :* [$\alpha$] = +14,4 (c=1%, MeOH, 20°C, $\lambda$=589nm)

**EXEMPLE 18 : Trans-1-{2-[(3,5-dibromobenzyl)oxy]-1,2,3,4-tétrahydro-naphthalén-1-yl}-1,4-diazépane, dichlorhydrate**

*Stade A : Trans-4-[2-hydroxy-1,2,3,4-tétrahydronaphthalén-1-yl]-1,4-diazépane-1-carboxylate de tert-butyle*

A température ambiante, on mélange 3,0g (20,5 mmoles) du composé obtenu au stade B de l'Exemple 15 et 4,1g (20,5 mmoles) de N-tert-butyloxycarbonyl homopipérazine dans 41 ml d'acétonitrile, puis on porte à reflux pendant 48h. Après évaporation et flash chromatographie sur 300g de silice (éluant : dichlorométhane/éthanol 98/2), on recueille le composé attendu.

*Stade B : Trans-4-{2-[(3, 5-dibromobenzyl)oxy]-1,2,3,4-tétrahydronaphthalén-1-yl}-1,4-diazépane-1-carboxylate de tert-butyle*

A température ambiante, sur 0,76g (19 mmoles) d'hydrure de sodium en suspension dans 20 ml de tétrahydrofurane, on ajoute en quelques minutes 6,0g (17,3 mmoles) du composé obtenu au stade précédent en solution dans 50 ml de tétrahydrofurane. On porte 2h à 50°C, puis ajoute d'un trait 5,7g (17,3 mmoles) de bromure de 3,5-dibromobenzyle, puis 6,4g (17,3 mmoles) d'iodure de tétra-n-butyl ammonium, et on porte la nuit à 70°C. On verse alors dans 200 ml d'eau et extrait par de l'éther éthylique. Les phases organiques jointes sont lavées et séchées. Après évaporation et flash chromatographie sur 500g de silice (éluant: dichlorométhane 100%, puis dichlorométhane/acétate d'éthyle 95/5, puis dichlorométhane/éthanol 95/5), on recueille le composé attendu.

*Stade C : Trans-1-{2-[(3,5-dibromobenzyl)oxy]-1,2,3,4-tétrahydro-naphthalén-1-yl}-1,4-diazépane, dichlorhydrate*

Sur 4,9g (8,2 mmoles) du composé obtenu au stade précédent en solution dans 250 ml d'acétate d'éthyle, à température ambiante, on fait buller doucement pendant quelques minutes de l'acide chlorhydrique gazeux. On maintient la nuit sous agitation à température ambiante, puis on concentre de moitié (apparition de précipité) et laisse agiter encore 2h. Après filtration, rinçage et séchage du précipité, on recueille le composé attendu sous forme de racémique.
*Point de fusion* (M.K.) : 112-118°C.

**EXEMPLE 19 : Isomère (-) du trans-1-{2-[(3,5-dibromobenzyl)oxy]-1,2,3,4-tétrahydro-naphthalén-1-yl}-1,4-diazépane, dichlorhydrate**

Après retour base, le composé racémique obtenu à l'Exemple 18 est séparé par chromatographie HPLC chirale préparative sur colonne CHIRALPAK AD (éluant : méthanol/diéthylamine 1000/1). Le premier des isomères ainsi séparés est salifié par chlorhydratation par l'éthanol chlorhydrique. Après filtration, rinçage et séchage, on obtient l'énantiomère (-) avec un excès énantiomérique supérieur à 98%.
*Point de fusion* (M.K.) : 122-126°C.

**EXEMPLE 20 : Isomère (+) du trans-1-{2-[(3,5-dibromobenzyl)oxy]-1,2,3,4-tétrahydro-naphthalén-1-yl}-1,4-diazépane, dichlorhydrate**

Le deuxième des isomères séparés à l' Exemple 19 est transformé en chlorhydrate, pour conduire au produit attendu.
*Point de fusion* (M.K.) : 119-140°C.

**EXEMPLE 21 : 1-{(1*S*,2*R*)-2-[(3,5-Dibromobenzyl)oxy]-2,3-dihydro-1*H*-indén-1-yl}pipérazine, dichlorhydrate**

*Stade A : (1S,2R)-1-{4-[(4-méthylphényl)sulfonyl]pipérazin-1yl)indan-2-ol*

A 8 g de (1*S*,2*R*)-1-aminoindan-2-ol (53,6 mmoles) en solution dans 80 ml de diméthylformamide sont ajoutés 15 ml de triéthylamine (107,2 mmoles) et 15,8 g de N,N-bis(2-chloroéthyl)-p-toluènesulfonamide (53,6 mmoles). On chauffe à 80°C pendant 24 heures, puis on évapore la diméthylformamide. Le résidu est repris au dichlorométhane, lavé, séché, filtré et évaporé. L'huile obtenue est purifiée par flash-chromatographie sur silice (éluant : dichlorométhane / acétate d'éthyle 95/5) pour conduire au produit attendu sous forme de meringue.

*Stade B : 1-{(1S,2R)-2-[(3,5-dibromobenzyl)oxy]-2,3-dihydro-1H-indén-1-yl}-4-[(4-méthylphényl)sulfonyl]pipérazine*

Le produit attendu est obtenu selon le procédé décrit au stade B de l'Exemple 1, à partir du composé obtenu au stade précédent.

*Stade C : 1-{(1S,2R)-2-[(3,5-dibromobenzyl)oxy]-2,3-dihydro-1H-indén-1-yl}pipérazine*

1,1 g du composé obtenu au stade précédent (1,77 mmole) sont chauffés 2 heures à 90°C en présence de 10 ml d'une solution de HBr/ acide acétique (33%) et de 808 mg d'acide 4-hydroxybenzoïque (5,85 mmoles). On refroidit, on ajoute NaOH (20%) jusqu'à pH = 10 puis on extrait au dichlorométhane, sèche, filtre et évapore. L'huile obtenue est purifiée par flash-chromatographie sur 100 g de silice (éluant : dichlorométhane / éthanol/ammoniaque 90/10/1) pour conduire au produit attendu sous forme d'huile incolore.

*Stade D : 1-{(1S,2R)-2-[(3,5-dibromobenzyl)oxy]-2,3-dihydro-1H-indén-1-yl}pipérazine, dichlorhydrate*

A 1,2 g du composé obtenu au stade précédent (2,57 mmoles) en solution dans l'acétate d'éthyle. On ajoute à température ambiante une solution 2M d'acide chlorhydrique dans l'éther. On agite 30 minutes puis évapore à sec. Le résidu est cristallisé dans l'acétate d'éthyle, filtré et séché pour conduire au produit attendu (composé cis) sous forme de cristaux blancs.
Point de fusion (MK) : 162-176°C
Pouvoir rotatoire [α]= - 2,9 (c=1%, MeOH, 20°C, λ=589nm)

**EXEMPLE 22 : 1-{(1*R*,2*S*)-2-[(3,5-Dibromobenzyl)oxy]-2,3-dihydro-1H-indén-1-yl}pipérazine, dichlorhydrate**

Le produit attendu est obtenu selon le procédé décrit dans l'Exemple 21, à partir de (1*R*,2*S*)-1-aminoindan-2-ol.
Point de fusion (MK) : 142-150°C.
Pouvoir rotatoire : [α] = + 5,8 (c=1%, MeOH, 20°C, λ=589nm)

**EXEMPLE 23 : Cis-1-{4-[(3,5-dibromobenzyl)oxy]-2,3,4,5-tétrahydro-1-benzoxépin-5-yl}pipérazine, dichlorhydrate**

*Stade A : 4-(4-Oxo-2,3,4,5-tétrahydro-1-benzoxépin-5-yl)pipérazine-1-carboxylate de tert-butyle*

A - 78°C, on coule 0,88 ml de chlorure d'oxalyle (10 mmoles) sur 25 ml de dichlorométane, puis, à la même température, on additionne lentement 1,02 ml de diméthylsulfoxyde (14,3 mmoles) . On laisse agiter 10 minutes à -78°C. Sur ce milieu réactionnel, on coule 2,5 g (7,17 mmoles) du composé obtenu au stade B de l'Exemple 8 en solution dans 15 ml de dichlorométane. On agite 15 minutes à -78°C puis on ajoute 5 ml (35,9 mmoles) de triéthylamine en 20 minutes. On laisse ensuite remonter le mélange à 0°C, puis on verse le milieu réactionnel sur 100 ml d'eau glacée. On extrait au dichlorométhane, sèche, filtre et évapore à sec pour conduire au produit attendu sous forme d'huile.

*Stade B : Cis-4-(4-hydroxy-2,3,4,5-tétrahydro-1-benzoxepin-5-yl)pipérazine-1-carboxylate de tert-butyle*

A 2,4 g (6,93 mmoles) du composé obtenu au stade précédent en solution dans 50 ml de méthanol sont ajoutés, à 0°C et par portions, 130 mg de $NaBH_4$ (3,5 mmoles, 0,5 équivalents). On agite 1 heure à 0°C puis évapore le méthanol. Le résidu obtenu est repris au toluène, lavé, séché, filtré et évaporé. Le résidu obtenu est purifié par flash-chromatographie sur silice (éluant : toluène/ éthanol 95/5) pour conduire au produit attendu sous forme d'huile.

*Stade C : Cis-1-{4-[(3,5-dibromobenzyl)oxy]-2,3,4,5-tétrahydro-1-benzoxépin-5-yl}pipérazine, dichlorhydrate*

Le produit attendu est obtenu selon le procédé décrit aux stades B et C de l'Exemple 1 à partir du composé obtenu au stade précédent.
Point de fusion (MK) : 166-191°C.

**EXEMPLE 24 : Cis-1-[2-((3,5-dibromobenzyloxy)-1,2,3,4-tétrahydronaphthalène-1-yl}pipérazine, dichlorhydrate**

Le produit attendu est obtenu selon le procédé décrit à l'Exemple 23, à partir du composé obtenu au stade C de l'Exemple 15.
Point de fusion (MK) :155-159°C.

**EXEMPLE 25 : Cis-1-{6-[(3,5-dibromobenzyl)oxy]-6,7,8,9-tétrahydro-5*H*-benzo[7]annulén-5-yl}pipérazine, dichlorhydrate**

*Stade A : 4-(6-Oxo-6,7,8,9-tétrahydro-5H-benzo[7]annulén-5-yl)pipérazine-1-carboxylate de tert-butyle*

A 4,4 g (12,6 mmoles) du composé obtenu au stade A de l'Exemple 7 en solution dans 44 ml de diméthylsulfoxyde sont ajoués, à température ambiante, 8,8g de forme stabilisée d' acide 2-iodoxybenzoïque (SIBX) (56,3 mmoles, 4,4 équivalents). On laisse agiter 2 heures à 25°C, puis on verse sur l'eau. L'insoluble obtenu est filtré. Le filtrat est extrait à l'acétate d'éthyle, séché, filtré et évaporé à sec. Le résidu obtenu est purifié par flash-chromatographie sur silice (éluant : dichlorométhane / acétate d'éthyle 95/5) pour conduire au produit attendu.
Point de fusion (BK) : 85-95°C.

*Stade B : 4-(6-Hydroxy-6,7,8,9-tétrahydro-5H-benzo[7]annulén-5-yl)pipérazine-1-carboxylate de tert-butyle*

1,5 g (4,3 mmoles) du composé obtenu au stade précédent sont traités selon la méthode décrite dans le stade B de l'Exemple 23. L'huile jaune obtenue est purifiée par flash-chromatographie sur silice (éluant : dichlorométhane / acétate d'éthyle 95/5) pour conduire au produit attendu sous forme de mélange cis/trans (rapport cis/trans = 80/20).

*Stade C : Cis-4-{6-[(3,5-dibromobenzyl)oxy]-6,7,8,9-tétrahydro-5H-benzo[7]annulén-5-yl}pipérazine-1-carboxylate de tert-butyle*

650 mg du composé obtenu au stade précédent (cis/trans = 80/20) (1,87 mmole) sont traités selon la méthode décrite dans le stade B de l'Exemple 1, pour conduire à une huile qui est purifiée par flash-chromatographie sur silice (éluant : cyclohexane/ acétate d'éthyle 95/5) pour conduire au produit attendu.

*Stade D : Cis-1-{6-[(3,5-dibromobenzyl)oxy]-6,7,8,9-tétrahydro-5H-benzo[7]annulén-5-yl}pipérazine, dichlorhydrate*

350 mg du composé obtenu au stade précédent (0,58 mmole) sont traités selon la méthode décrite dans le stade C de l'Exemple 1, puis le produit obtenu est cristallisé dans l'éther isopropylique pour conduire au produit attendu sous forme de cristaux blancs.
*Point de fusion* (MK) : 149-158°C.

**EXEMPLE 26 : Trans-3-[(3,5-dibromobenzyl)oxy]-4-pipérazin-1-ylchromane-6-carbonitrile, dichlorhydrate**

*Stade A : 6-Bromo-1a,7b-dihydro-2H-oxiréno[c]chromène*

8,5 g de 6-bromo-2*H*-chromène (40,3 mmoles) préparés selon la synthèse décrite dans J. Org. Chem.,1998, 63 , 864 sont traités selon la méthode décrite dans le stade A de l'Exemple 8, pour conduire au produit attendu sous forme d'huile.

*Stade B : Trans-4-(6-bromo-3-hydroxy-3,4-dihydro-2H-chromén-4-yl)pipérazine-1-carboxylate de tert-butyle*

7,6 g du composé obtenu au stade précédent (33,5 mmoles) sont traités selon la méthode décrite dans le stade A de l'Exemple 1. Le résidu obtenu est purifié par flash-chromatographie sur silice (éluant : toluène/ éthanol 95/5) pour conduire au produit attendu sous forme d'huile.

*Stade C : Trans-4-(6-cyano-3-hydroxy-3,4-dihydro-2H-chromén-4-yl)pipérazine-1-carboxylate de tert-butyle*

1 g du composé obtenu au stade précédent (2,4 mmoles) sont mis en solution dans 10 ml de diméthylformamide. La solution est dégazée à l'argon puis on ajoute 112 mg de palladium (0) tétrakis triphénylphosphine (0,09 mmole) et 170 mg de cyanure de zinc (1,4 mmole). On chauffe à 80°C pendant 3 jours. Puis le milieu réactionnel est refroidi et versé sur l'eau. On extrait à l'acétate d'éthyle, lave à l'eau, sèche, filtre et évapore à sec. Le résidu obtenu est purifié par flash-chromatographie sur silice (éluant : dichlorométhane/ acétate d'éthyle 90/10) pour conduire au produit attendu sous forme d'huile.

*Stade D : Trans-3-[(3,5-dibromobengyl)oxy]-4-piperazin-1-ylchromane-6-carbonitrile, dichlorhydrate*

Le produit attendu est obtenu selon le procédé décrit aux stades B et C de l'Exemple 1, à partir du composé obtenu au stade précédent.
*Point de fusion* (MK) : 185-200°C.

**EXEMPLE 27 : Trans-7-[(3,5-dibromobenzyl)oxy]-8-pipérazin-1-yl-5,6,7,8-tétrahydronaphthalène-2-carbonitrile, dichlorhydrate**

*StadeA : 6-Bromo-1a,2,3,7b-tétrahydronaphtho[1,2-b]oxirène*

7,4 g du composé de la Préparation A (35 mmoles) sont traités par l'Oxone® selon la méthode décrite dans le stade A de l'exemple 8, pour conduire au produit attendu sous forme d'huile.

*<u>Stade B</u> : Trans-4-(7-bromo-2-hydroxy-1,2,3,4-tétrahydronaphthalén-1-yl)-pipérazine-1-carboxylate de tert-butyle*

On solubilise 6,5 g de pipérazine-1-carboxylate de *tert*-butyle (35 mmoles) et 7,9 g du composé obtenu au stade précédent (35 mmoles) dans 45 ml de diméthylformamide, puis on chauffe le mélange réactionnel à 110°C pendant 24 heures. Après évaporation à sec, on obtient un résidu qui est purifié par flash-chromatographie sur silice (éluant : toluène/ éthanol 98/2), pour conduire au produit attendu sous forme d'huile.

*<u>Stade C</u> : Trans-4-(7-cyano-2-hydroxy-1,2,3,4-tétrahydronaphthalén-1-yl)-pipérazine-1-carboxylate de tert-butyle*

4 g du composé obtenu au stade précédent (9,7 mmoles) sont traités selon la méthode décrite dans le stade C de l'Exemple 26. On chauffe à 80°C pendant 2 heures.
Puis le milieu réactionnel est refroidi et versé sur l'eau. On extrait au dichlorométhane, lave à l'eau, sèche, filtre et évapore à sec. Le résidu obtenu est purifié par flash-chromatographie sur silice (éluant : dichlorométhane/ éthanol 98/2) pour conduire au produit attendu sous forme d'huile.

*<u>Stade D</u> : Trans-7-[(3,5-dibromobenzyl)oxy]-8-pipérazin-1-yl-5,6,7,8-tétrahydronaphthalène-2-carbonitrile, dichlorhydrate*

Le produit attendu est obtenu selon le procédé décrit aux stades B et C de l'Exemple 1, à partir du composé obtenu au stade précédent.
*<u>Point de fusion</u>* (MK) : 169-191°C.

**<u>EXEMPLE 28 :</u> Trans-4-[(3,5-dibromobenzyl)oxy]-5-pipérazin-1-yl-2,3,4,5-tétrahydro-1-benzoxépine-7-carbonitrile, dichlorhydrate**

*<u>Stade A</u> : 7-Bromo-1a,2,3,8b-tétrahydrooxireno[d][1]benzoxépine*

4 g du composé de la Préparation B sont traités par l'Oxone® selon la méthode décrite dans le stade A de l'Exemple 8, pour conduire au produit attendu sous forme d'huile.

*<u>Stade B</u> : Trans-4-(7-bromo-4-hydroxy-2,3,4,5-tétrahydro-1-benzoxépin-5-yl)pipérazine-1-carboxylate de tert-butyle*

3,9 g du composé obtenu au stade précédent (16,2 mmoles) sont traités selon la méthode décrite dans le stade A de l'Exemple 1. Le chauffage est prolongé 3 jours. On obtient ainsi un résidu qui est purifié par flash-chromatographie sur silice (éluant : dichlorométhane/éthanol 98/2) pour conduire au produit attendu sous forme d'huile.

*<u>Stade C</u> : Trans-4-(7-cyano-4-hydroxy-2,3,4,5-tétrahydro-1-benzoxépin-5-yl)pipérazine-1-carboxylate de tert-butyle*

5,8 g du composé obtenu au stade précédent (13,6 mmoles) sont traités selon la méthode décrite dans le stade C de l'Exemple 26. On chauffe à 80°C pendant 20h. Puis le milieu réactionnel est refroidi et versé sur l'eau. On extrait au dichlorométhane, lave à l'eau, sèche, filtre et évapore à sec. Le résidu obtenu est purifié par flash-chromatographie sur silice (éluant : toluène/ éthanol 95/5) pour conduire au produit attendu sous forme d'huile.

*<u>Stade D</u> : Trans-4-[(3,5-dibromobenzyl)oxy]-5-pipérazin-1-yl-2,3,4,5-tétrahydro-1-benzoxépine-7-carbonitrile, dichlorhydrate*

Le produit attendu est obtenu selon le procédé décrit aux stades B et C de l'Exemple 1, à partir du composé obtenu au stade précédent.
*<u>Point de fusion</u>* (MK) : 163-195°C.

**<u>EXEMPLE 29</u> : Trans-5-[(4-{2-[(3,5-dibromobenzyl)oxy]-2,3-dihydro-1*H*-indén-1-yl}pipérazin-1-yl)méthyl]-2,4-dihydro-3*H*-1,2,4-triazol-3-one, dichlorhydrate**

*<u>Stade A</u> : Trans-5-[(4-{2-[(3,5-dibromobenzyl)oxy]-2,3-dihydro-1H-indén-1-yl}pipérazin-1-yl)méthyl]-2,4-dihydro-3H-1,2,4-triazol-3-one*

Après retour base, le composé obtenu au stade C de l'Exemple 1 (2,14 mmoles), est solubilisé dans 30 ml de diméthylformamide. On ajoute 0,75 ml (4,28 mmoles) de diisopropyléthylamine et 314 mg (2,35 mmoles) de 5-(chlorométhyl)-2,4-dihydro-3*H*-1,2,4-triazol-3-one préparée selon la méthode décrite dans Tetrahedron Letters, 2000, 41, 8661. On agite 12 heures à température ambiante, puis on évapore à sec. Le résidu obtenu est repris par du dichlorométhane, lavé, séché, filtré et évaporé. L'huile obtenue est purifiée par flash-chromatographie sur silice pour conduire au produit attendu.

*Stade B : Trans-5-[(4-{2-[(3,5-dibromobenzyl)oxy]-2,3-dihydro-1H-indén-1-yl}pipérazin-1-yl)méthyl]-2,4-dihydro-3H-1,2,4-triazol-3-one, dichlorhydrate*

800 mg du composé obtenu au stade précédent (1,42 mmole) sont mis en solution dans 50 ml d'acétate d'éthyle. Puis on ajoute 2,8 ml (5,68 mmoles) d'une solution 2M d'acide chlorhydrique dans l'éther. Après agitation 30 minutes à température ambiante, le mélange est évaporé à sec et le résidu cristallisé dans l'acétonitrile, pour conduire au produit attendu.
*Point de fusion* (MK) : 198-202°C.

**EXEMPLE 30 : Trans-1-{2-[(3,5-dibromobenzyl)oxy]-2,3-dihydro-1*H*-indén-1-yl}-4-[(2*S*)-2,3-dihydro-1,4-benzodioxin-2-ylméthyl]pipérazine, dichlorhydrate**

*Stade A : 4-[(2R)-2,3-dihydro-1,4-benzodioxin-2-ylcarbonyl]pipérazine-1-carboxylate de tert-butyle*

5 g d'acide (2*R*)-2,3-dihydro-1,4-benzodioxine-2-carboxylique (27,8 mmoles) sont mis en solution dans 300 ml d'acétonitrile. On ajoute 6 g de NN'-dicyclohexylcarbodiimide (29,1 mmole) puis 4,1 g de 1-hydroxybenzotriazole (30,6 mmoles). Enfin, on additionne 6,2 g de pipérazine-1-carboxylate de *tert*-butyle (33,4 mmoles) et on agite 12 heures à température ambiante. Le milieu réactionnel est ensuite filtré, puis le filtrat évaporé à sec.

L'huile ainsi obtenue est purifiée par flash-chromatographie sur silice (éluant : toluène/acétate d'éthyle 80/20) pour conduire au produit attendu sous forme d'huile.

*Stade B : 4-[(2S)-2,3-dihydro-1,4-benzodioxin-2-ylméthyl]pipérazine-1-carboxylate de tert-butyle*

500 mg du composé obtenu au stade précédent (1,44 mmole) sont mis en solution dans 5 ml de tétrahydrofurane anhydre. On ajoute, goutte à goutte, à température ambiante, 4,4 ml (4,3 mmoles) d'une solution 1 M de borane dans le tétrahydrofurane. On chauffe au reflux 2 heures. On refroidit et on hydrolyse lentement avec 5 ml d'éthanol.
On évapore à sec. Le résidu obtenu est cristallisé dans l'eau, filtré et séché, pour conduire au produit attendu.
*Point de fusion* (BK) = 97-99°C.

*Stade C : 1-[(2S)-2,3-Dihydro-1,4-benzodioxin-2-ylméthyl]pipérazine, dichlorhydrate*

7,5 g du composé obtenu au stade précédent (22,4 mmoles) sont agités 2 jours, à température ambiante, en présence de 100 ml d'une solution 2,6 N d'acide chlorhydrique dans l'éthanol. Les cristaux formés sont filtrés et séchés, pour conduire au produit attendu sous forme d'un solide blanc.
*Point de fusion* (BK) = 166-172°C.

*Stade D : Trans 1-{4-[(2S)-2,3-dihydro-1,4-benzodioxin-2 ylméthyl]pipérazin-1-yl}indan-2-ol*

A 1,6 g (6,8 mmoles) de 1-[(2S)-2,3-dihydro-1,4-benzodioxin-2-ylméthyl]pipérazine, obtenue par retour base du composé du stade précédent, en solution dans 10 ml d'acétonitrile sont ajoutés 1,08 g d'indène oxyde (8,16 mmoles). Le mélange réactionnel est chauffé à 80°C pendant 30 heures, puis évaporé à sec. L'huile ainsi obtenue est purifiée par flash-chromatographie sur silice (éluant : dichlorométhane/ éthanol/ ammoniaque 90/10/1) pour conduire au composé attendu sous forme de meringue.

*Stade E: Trans-1-{2-[(3,5-dibromobenzyl)oxy}-2,3-dihydro-1H-indén-1-yl}-4-[(2S)-2,3-dihydro-1,4-benzodioxin-2-ylméthyl]pipérazine*

Le produit attendu est obtenu selon le procédé décrit au stade B de l'Exemple 1, à partir du composé obtenu au stade précédent.

*Stade F : Trans-1-{2-[(3,5-dibromobenzyl)oxy]-2,3-dihydro-1H-indén-1-yl}-4-[(25)-2,3-dihydro-1,4-benzodioxin-2 ylméthyl]pipérazine, dichlorhydrate*

A 210 mg du composé obtenu au stade précédent (0,34 mmole) en solution dans 20 ml d'acétate d'éthyle sont ajoutés à température ambiante 0,5 ml (1 mmole) d'une solution 2M d'acide chlorhydrique dans l'éther. Après 30 minutes d'agitation, le mélange réactionnel est évaporé à sec. Le résidu obtenu est cristallisé dans l'acétate d'éthyle, filtré et séché pour conduire au produit attendu sous forme de cristaux blancs.
Point de fusion (MK) : 137-145°C.

**EXEMPLE 31 : (+) Cis-1-{(1*S*,2*R*)-2-[(3,5-dibromobenzyl)oxy]-2,3-dihydro-1*H*-indén-1-yl}-4-(pyridin-2-ylcarbonyl)pipérazine, dichlorhydrate**

*Stade A : Cis 1-{(1S,2R)-2-[(3,5-dibromobenzyl)oxy]-2,3-dihydro-1H-indén-1-yl}-4-(pyridin-2-ylcarbonyl)pipérazine*

A 263 mg d'acide pyridine-2-carboxylique (2,14 mmole) en solution dans 10 ml de tétrahydrofurane sont ajoutés 382 mg de carbonyldiimidazole (2,35 mmole, 1,1 équivalent), puis, après 3 heures d'agitation à température ambiante, une solution de 1 g (2,14 mmole) du composé obtenu au stade C de l'Exemple 21, dans 20 ml de tétrahydrofurane. On agite 12 heures à 25°C, puis on ajoute de l'eau, extrait au dichlorométhane, sèche, filtre et évapore à sec. L'huile obtenue est purifiée par flash-chromatographie sur silice (éluant : dichlorométhane/ éthanol 95/5) pour conduire au produit attendu sous forme d'huile.

*Stade B : Cis-1-{(1S,2R)-2-[(3,5-dibromobenzyl)oxy]-2,3-dihydro-1H-indén-1-yl}-4-(pyridin-2-ylcarbonyl)pipérazine, dichlorhydrate*

900 mg du composé obtenu au stade précédent (1,57 mmole) sont traités selon la méthode décrite au stade F de l'Exemple 30. Le solide obtenu est filtré et séché, pour conduire au produit attendu sous forme de cristaux blanc.
Point de fusion (MK) : 108-122°C.
Pouvoir rotatoire : [$\alpha$] = + 27,3 (c=1%, méthanol, 20°C, $\lambda$=589nm)

**EXEMPLE 32 : 1-(Trans-2-{1-[3,5-bis(trifluorométhyl)phényl]éthoxy}-1,2,3,4-tétrahydro-naphthalén-1-yl)pipérazine dichlorhydrate, diastéréoisomère 1**

*Stade A : 4-(Trans-2-{[3,5-bis(trifluorométhyl)benzoyl]oxy}-1,2,3,4-tétrahydronaphthalén-1-yl)pipérazine-1-carboxylate de tert-butyle*

A 11,7g (35,2 mmoles) du composé obtenu au stade C de l'Exemple 15 et 5,9 ml (42,2 mmoles) de triéthylamine en solution dans 300 ml de dichlorométhane, sont ajoutés à température ambiante 7,05 ml (38,7mmoles) de chlorure de bis(trifluorométhyl)benzoyle dans 50 ml de dichlorométhane, goutte à goutte en 1h10, puis 0,5g de diméthylaminopyridine. Le mélange réactionnel est ensuite porté à reflux pendant 5 jours, puis 1,3 ml supplémentaires du chlorure d'acide sont ajoutés, et le reflux est poursuivi pendant 20h. Après évaporation du milieu, le résidu obtenu est filtré sur 200g de silice (éluant : dichlorométhane), pour conduire au produit attendu.

*Stade B : 4-[Trans-2-({1-[3,5-bis(trifluorométhyl)phényl]vinyl}oxy)-1,2,3,4-tétrahydronaphthalén-1-yl]pipérazine-1-carboxylate de tert-butyle*

A 13,2g (23 mmoles) du composé obtenu au stade précédent dans 92 ml de tétrahydrofurane sont ajoutés à température ambiante 46 ml d'une solution molaire de dicyclopentadiényl diméthyl titane dans le toluène, puis le mélange réactionnel est porté à 85°C pendant 20h. On ajoute alors 23 ml supplémentaires de la solution molaire de dicyclopentadiényl diméthyl titane dans le toluène, goutte à goutte en 10 mn à 85°C, puis poursuit à cette température pendant encore 24h. On laisse refroidir, ajoute 500 ml de pentane, filtre sur célite et rince au pentane jusqu'à décoloration du filtrat. Après évaporation de tous les filtrats joints et flash chromatographie sur 800g de silice (éluant : dichlorométhane, puis dichlorométhane/acétate d'éthyle 98/2), on obtient le produit attendu.

*Stade C : 4-(Trans-2-{1-[3,5-bis(trifluorométhyl)phényl]éthoxy}-1,2,3,4-tétrahydronaphthalén-1-yl)pipérazine-1-carboxylate de tert-butyle*

8,34g (14,6 mmoles) du composé obtenu au stade précédent en solution dans 150 ml d'éthanol sont hydrogénés à température ambiante et pression atmosphérique pendant 7h en présence de 1g de palladium à 5% sur charbon.

Après filtration sur célite, rinçage à l'éthanol et évaporation, on obtient le produit attendu sous la forme d'un mélange de diastéréoisomères non quantifiables et non séparables.

*Stade D : 1-(Trans-2-{1-[3,5-bis(trifluorométhyl)phényl]éthoxy}-1,2,3,4-tétrahydronaphthalén-1-yl)pipérazine dichlorhydrate, diastéréoisomère 1*

Sur 8,4g (14,6 mmoles) du composé obtenu au stade précédent en solution dans 500 ml d'acétate d'éthyle, on fait buller doucement à température ambiante pendant quelques minutes de l'acide chlorhydrique gazeux. On maintient la nuit sous agitation à température ambiante, puis évapore à sec. Le résidu est solubilisé dans 100 ml d'eau et, sous agitation énergique, on basifie par 8g de carbonate de sodium. La pâte qui relargue est extraite par 2 fois 100 ml de dichlorométhane. Les phases organiques jointes sont séchées et concentrées, et le résidu obtenu est chromatographié sur 700g de silice (éluant : dichlorométhane/éthanol/ammoniaque 95/5/0,5) pour conduire au premier diastéréoisomère qui est transformé en son dichlorhydrate par action de l'éther chlorhydrique. Après cristallisation dans le pentane, on obtient le produit attendu.
*Point de fusion* (M.K.) : 98-102°C.

**EXEMPLE 33 : 1-(Trans-2-{1-[3,5-bis(trifluorométhyl)phényl)éthoxy}-1,2,3,4-tétrahydro-naphthalén-1-yl)pipérazine dichlorhydrate,** diastéréoisomère 2

Le second produit élué au stade D de l'Exemple 32 conduit au second diastéréoisomère, qui est transformé en son dichlorhydrate.
*Point de fusion* (M.K.) : 98-101°C.

**EXEMPLE 34 : 1-[(1S,2R)-2-Benzyloxy-2,3-dihydro-1*H*-indén-1-yl]-pipérazine, dichlorhydrate**

Le produit attendu (composé cis) est obtenu selon le procédé de l'Exemple 21, en remplaçant au stade B le bromure de 3,5-dibromobenzyle par le bromure de benzyle.
*Point de fusion* : 104-125°C.

**EXEMPLE 35 : Trans-1-{2-[(3,5-diméthylbenzyl)oxy]-2,3-dihydro-1*H*-indén-1-yl}pipérazine, dichlorhydrate**

Le produit attendu est obtenu selon le procédé décrit aux stades D et E de l'Exemple 15, à partir du composé obtenu au stade A de l'Exemple 1 et de bromure de 3,5-diméthylbenzyle.
*Point de fusion* : 161-70°C.

**EXEMPLE 36 : 1-[(1S,2R)-2-[(3,5-Difluorobenzyl)-oxy]-2,3-dihydro-1*H*-indén-1-yl]-pipérazine, dichlorhydrate**

Le produit attendu (composé cis) est obtenu selon le procédé de l'Exemple 21, en remplaçant au stade B le bromure de 3,5-dibromobenzyle par le bromure de 3,5-difluorobenzyle.
*Point de fusion* : 160-190°C.

**EXEMPLE 37 : 1-[(1S,2R)-2-Benzyloxy-2,3-dihydro-1*H*-indén-1-yl]-4-méthylpipérazine, dichlorhydrate**

Le produit attendu est obtenu par N-méthylation du composé de l'Exemple 34.
*Point de fusion* : 182-189°C.

**EXEMPLE 38 : 1-[(1S,2R)-2-[(3,5-Diméthylbenzyl)-oxy]-2,3-dihydro-1*H*-indén-1-yl]-pipérazine, dichlorhydrate**

*Stade A : 1-{(1S,2R)-2-[(3,5-Diméthylbenzyl)oxy]-2,3-dihydro-1H-indén-1-yl}-4-[(4-méthylphényl) sulfonyl] pipérazine*

Le produit attendu est obtenu selon le procédé décrit aux stades A et B de l'Exemple 21, en remplaçant au stade B le bromure de 3,5-dibromobenzyle par le bromure de 3,5-diméthylbenzyle.

*Stade B : 1-{(1S,2R)-2-[(3,5-Diméthylbenzyl)oxy]-2,3-dihydro-1H-indén-1yl}-pipérazine*

A une solution de 3,6 g de naphthalène (28 mmoles) dans 30 ml de 1,2-diméthoxyéthane, sont ajoutés 3 g de sodium (130 mmoles). Le milieu réactionnel est agité à température ambiante pendant 2 heures pour former la solution de sodium/naphthalène/1,2-diméthoxyéthane. 14,8 ml (64 mmoles) de cette solution sont ajoutés à une température

de -70°C à une solution de 3g (6,1 mmoles) du composé obtenu au stade précédent dans 55 ml de 1,2-diméthoxyéthane. La couleur de la solution passe du blanc au bleu. On agite 30 minutes à -70°C, puis on hydrolyse par ajout de 100 ml d'eau. On extrait alors à l'acétate d'éthyle, sèche, filtre et évapore à sec. Le résidu obtenu est purifié par flash-chromatographie sur 200g de silice (éluant : dichlorométhane/ éthanol/ ammoniaque 90/10/1) pour conduire au produit attendu sous forme d'huile.

*Stade C : 1-{(1S,2R)-2-[(3,5-Diméthylbenzyl)oxy]-2,3-dihydro-1H-indén-1-yl)-pipérazine, dichlorhydrate*

A 1,2 g (3,58 mmoles) du composé obtenu au stade précédent dans 50 ml d'acétonitrile sont ajoutés 3,6 ml (7,16 mmoles, 2 équivalents) d'une solution d'éther chlorhydrique 2M. On amorce la cristallisation en grattant, puis on laisse agiter 15 minutes à température ambiante. Les cristaux blanc obtenus sont séchés, pour conduire au produit attendu.
*Point de fusion* : 170-193°C.

**EXEMPLE 39 : Trans-1-{2-[(3,5-difluorobenzyl)oxy]-2,3-dihydro-1*H*-indén-1-yl}pipérazine, dichlorhydrate**

Le produit attendu est obtenu selon le procédé l'Exemple 1, en remplaçant au stade B le bromure de 3,5-dibromobenzyle par le bromure de 3,5-difluorobenzyle.
*Point de fusion :* 185-198°C.

**EXEMPLE 40 : Trans-1-(2-{[3,5-bis(trifluorométhyl)benzyl]oxy}-2,3-dihydro-1*H*-indén-1-yl)pipérazine, dichlorhydrate**

Le produit attendu est obtenu selon le procédé l'Exemple 1, en remplaçant au stade B le bromure de 3,5-dibromobenzyle par le bromure de 3,5-bis(trifluorométhyl)benzyle.
*Point de fusion* : 140-160°C.

**EXEMPLE 41 : Trans-1-{2-[(3,5-dibromobenzyl)oxy]-6-méthoxy-2,3-dihydro-1*H* indén-1-yl}pipérazine, dichlorhydrate**

Le produit attendu est obtenu selon le procédé de l'Exemple 4, en remplaçant au stade A le 2*H*-chromène par le 5-méthoxy-1*H*-indène.
*Point de fusion* : 184-195°C.

**EXEMPLE 42 : Trans-1-{2-[(3,5-dichlorobenzyl)oxy]-6-méthoxy-2,3-dihydro-1*H*-indén-1-yl}pipérazine, dichlorhydrate**

Le produit attendu est obtenu selon le procédé de l'Exemple 4, à partir de 5-méthoxy-1*H*-indène et de bromure de 3,5-dichlorobenzyle.
*Point de fusion* : 169-176°C.

**EXEMPLE 43 : Trans-1-{2-[(3,5-dichlorobenzyl)oxy)-2,3-dibydro-1*H*-indén-1-yl}pipérazine, dichlorhydrate**

Le produit attendu est obtenu selon le procédé de l'Exemple 1, en remplaçant au stade B le bromure de 3,5-dibromobenzyle par le bromure de 3,5-dichlorobenzyle.
*Point de fusion* : 115-127°C.

**EXEMPLE 44 : Trans-1-{3-[(3,5-dichlorobenzyl)oxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3,5-dichlorobenzyle.
*Point de fusion :* 110-118°C.

**EXEMPLE 45 : Trans-1-{2-[(3,5-bis(trifluorométhyl)benzyl)oxy]-1,2,3,4-tétrahydronaphthalén-1-yl}pipérazine, dichlorhydrate**

Le produit attendu est obtenu selon le procédé de l'Exemple 15, en remplaçant au stade D le bromure de 3,5-dibromobenzyle par le bromure de 3,5-bis(trifluorométhyl)benzyle.

*Point de fusion* (MK) : 98-101°C.

**EXEMPLE 46 : Trans-1-{3-[3-fluoro-5-(trifluorométhyl)benzyloxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3-fluoro-5-(trifluorométhyl)benzyle.
*Point de fusion :* 102-113°C.

**EXEMPLE 47 : Trans-1-{3-(3-chloro-5-fluorobenzyloxy)-3,4-dihydro-2H-chromén-4-yl}pipérazine, dichlorhydrate**

*Stade A : Trans-4-{3-[(3-chloro-5-fluorobenzyl)oxy]-3,4-dihydro-2H-chromén-4-yl] pipérazine-1- carboxylate de tert-butyle*

Le produit attendu est obtenu selon le procédé décrit aux stades A à D de l'Exemple 4, en remplaçant au stade D le bromure de 3,5-dibromobenzyle par le bromure de 3-chloro-5-fluorobenzyle.

***Stade B*** *: Trans-1-{3-(3-chloro-5-fluorobenzyloxy)-3,4-dihydro-2H-chromén-4-yl}pipérazine, dichlorhydrate*

Le produit attendu est obtenu selon le procédé décrit au stade C de l'Exemple 1, à partir du composé obtenu au stade précédent.
*Point de fusion :* 90-95°C.

**EXEMPLE 47 bis : Trans-1-{3-(3-chloro-5-fluorobenzyloxy)-3,4-dihydro-2H-chromén-4-yl}pipérazine, diméthanesulfonate**

Le produit attendu est obtenu par réaction du composé de l'Exemple 47 avec la soude, suivie de la salification du produit ainsi obtenu par l'acide méthanesulfonique.
*Point de fusion :* 161-171°C.

**EXEMPLE 48 : Cis-4-[2-(3,5-dibromobenzyloxy)-1,2,3,4-tétrahydronaphtalén-1-yl]morpholine, chlorhydrate**

Le produit attendu est obtenu selon le procédé de l'Exemple 23, en remplaçant au stade A le composé obtenu au stade B de l'Exemple 8, par le composé obtenu au stade B de l'Exemple 10.
*Point de fusion :* 195-198°C.

**EXEMPLE 49 : Trans-4-{3-[(3,5-dibromobenzyl)oxy]-3,4-dihydro-2*H*-chromén-4-yl}morpholine, chlorhydrate**

Le produit attendu est obtenu selon le procédé décrit aux stades B à D de l'Exemple 10, à partir du composé obtenu au stade B de l'Exemple 4.
*Point de fusion :* 143-148°C.

**EXEMPLE 50 : Trans-4-{2-[(3,5-dibromobenzyl)oxy]-1,2,3,4-tétrahydro-1-naphthalényl}pipéridine, chlorhydrate**

*Stade A. 1-(4-Pyridinyl)-1,2,3,4-tétrahydro-1-naphthalénol*

Sur une solution de 20g de 4-bromopyridine dans 73 mL d'éther, refroidie à -78°C, on coule goutte à goutte 85ml d'une solution 1,5M dans l'hexane de n-butyllithium. On agite 30 minutes à cette température, puis coule une solution de 1-tétralone dans 73ml d'éther et laisse remonter à température ambiante à la fin de l'addition. Après une nuit sous agitation, on coule une solution aqueuse saturée en chlorure d'ammonium. On décante, extrait à l'éther. On réunit les phases éthérées, les extrait à l'acide chlorhydrique 1N. Les phases acides réunies sont amenées à pH=8 par de la soude à 20%, extraites au dichlorométhane. Après séchage, on isole le produit attendu qui est purifié par chromatographie rapide sur silice (éluant : dichlorométhane/ méthanol 95/5) pour conduire au produit attendu.
*Point de fusion* (B.K.) = 160-162°C.

*<u>Stade B:</u> 4-(3,4-Dihydro-1-naphthalényl)pyridine*

On mélange 2g du composé obtenu au stade précédent, 10ml d'eau et 10ml d'acide sulfurique à 95%, chauffe 30 minutes à 80°C, refroidit à 0°C et amène à pH=10 par de la soude à 20%. On extrait au dichlorométhane, lave à l'eau, sèche et évapore pour conduire au produit attendu.

*<u>Stade C :</u> 4-(2,3-Dihydronaphto[1,2-b]oxiren-7b(1aH)-yl)pyridine 1-oxyde*

A une température comprise entre 20 et 25°C, on coule une solution composée de 5,4g du produit obtenu au stade précédent, 20g de bicarbonate de soude, 35ml d'acétone, 20 ml d'eau et 200ml d'acétate d'éthyle sur une solution de 29,3 g d'Oxone® dans 200ml d'eau. On agite une nuit à température ambiante, dilue à l'eau et extrait à l'acétate d'éthyle. Après traitement habituel, on isole le produit attendu.

***<u>Stade D</u>** <u>:</u> 1-(1-Oxydo-4-pyridinyl)-1,2,3,4-tétrahydro-2-naphthalénol*

Sur une solution de 450 mg du produit obtenu au stade précédent dans 10 ml de tétrahydrofurane anhydre, en présence d'une trace de vert de bromocrésol, on ajoute en une fois et à température ambiante, 289 mg de cyanoborohydrure de sodium. On additionne de l'éthérate de trifluorure de bore jusqu'à ce que l'indicateur coloré vire au jaune et autant de fois qu'il le faut au cours de la réaction pour maintenir le pH à 4-5. A la fin de la réaction, on ajoute de l'acide chlorhydrique concentré jusqu'à pH=1 et on agite 30 minutes à température ambiante. On amène ensuite à pH=8 par de la soude, extrait à l'acétate d'éthyle et après traitement on isole le produit attendu (80% trans, 20% cis).

*<u>Stade E :</u> 1-(4-Pipéridinyl)-1,2,3,4-tétrahydro-2-naphthalénol*

On mélange dans un réacteur 1,78 g du produit obtenu au stade précédent, 1g d'oxyde de platine, 0,75 ml d'acide chlorhydrique concentré, 75ml d'éthanol et hydrogène sous une pression de 1 bar. Après 6 h de réaction à température ambiante, on filtre, ajoute 8ml de soude, évapore l'éthanol, solubilise dans le minimum d'eau et ajuste le pH à 10. Après extraction et traitement habituel, on isole le produit attendu (80% trans, 20% cis).

*<u>Stade F:</u> 4-(2-Hydroxy-1,2,3,4-tétrahydro-1-naphthalényl)-1-pipéridinecarboxylate de tert-butyle*

Une solution de 2,1 g de dicarbonate de di-(tert-butyle) dans 50 ml de dichlorométhane est ajoutée sur 2,06g du produit obtenu au stade précédent en solution dans 50 ml de dichlorométhane. On laisse deux heures sous agitation à température ambiante puis évapore à sec. Après purification sur silice (éluant : dichlorométhane/ méthanol 95/5), on isole le produit attendu (80% trans, 20% cis).

*<u>Stade G :</u> 4-{2-[(3,5-Dibromobenryl)oxy]-1,2,3,4-tétrahydro-1-naphthalényl}-1-pipéridinecarboxylate de tert-butyle*

On introduit 241 mg d'hydrure de sodium, à 60% dans l'huile, sur une solution de 1,9 g du composé obtenu au stade précédent dans 20 ml de tétrahydrofurane anhydre, refroidie à 0°C. On agite 15 minutes, puis on ajoute, toujours à cette température, 20 mg d'iodure de tétrabutylammonium et enfin 1,9g de bromure de 3,5-dibromobenzyle. On laisse remonter à température ambiante et agite 24h. On évapore à sec, reprend à l'eau et au dichlorométhane et après traitement habituel et chromatographie sur silice (éluant dichlorométhane), on isole le produit attendu sous forme d'une meringue blanche (80% trans, 20% cis).

*<u>Stade H</u> : Trans-4-{2-[(3,5-dibromobenzyl)oxy]-1,2,3,4-tétrahydro-1-naphthalényl}-pipéridine, chlorhydrate*

2 g du composé obtenu au stade précédent, dans 20 ml d'éthanol est traité par 19 ml d'une solution 3,6 N d'éthanol chlorhydrique. Après 24h, on filtre le précipité formé, le rince et le sèche pour conduire au produit attendu sous forme de chlorhydrate. (Le composé cis se trouve dans le filtrat).
*<u>Point de fusion</u>* : 152-167°C.

**<u>EXEMPLE 51</u> : Cis-1-acétyl-3-[(3,5-dibromobenzyl)oxy]-4-(1-pipérazinyl)-1,2,3,4-tétrahydroquinoléine, dichlorhydrate**

*<u>Stade A</u> : 1-Acétyl-1,2-dihydroquinoléine*

Dans une solution de 20g de quinoléine dans 200ml d'acide acétique et 77,5ml d'anhydride acétique, refroidie

à 0°C, on introduit par portions 23,43g de borohydrure de sodium. On chauffe ensuite 2h à 60°C puis laisse agiter la nuit à température ambiante. On concentre, dilue à l'eau, et amène à pH = 10 avec de la soude, extrait à l'éther. Les phases éthérées réunies sont lavées avec de l'acide chlorhydrique 1N, puis à pH neutre et après traitement habituel, on isole le produit attendu.

*Stade B : 3-Acétyl-1a,2,3,7b-tétrahydrooxiréno[2,3-c]quinoléine*

Le produit attendu est obtenu selon le procédé décrit au stade A de l'Exemple 8, à partir du composé obtenu au stade précédent.

*Stade C : Trans-4-[1-acétyl-3-hydroxy-1,2,3,4-tétrahydro-4-quinoléinyl]-1-pipérazinecarboxylate de tert-butyle*

Le produit attendu est obtenu selon le procédé décrit au stade A de l'Exemple 1, à partir du composé obtenu au stade précédent.

*Stade D : Cis-1-acétyl-3-[(3,5-dibromobenzyl)oxy]-4-(1-pipérazinyl)-1,2,3,4-tétrahydroquinoléine, dichlorhydrate*

Le produit attendu est obtenu selon le procédé de l'Exemple 23, à partir du composé obtenu au stade précédent.
Point de fusion : 164-167°C.

**EXEMPLE 52 : Trans-1-{3-[(3,5-dibromobenzyl)oxy]-3,4-dihydro-2*H*-chromén-4-yl}-N-méthyl-4-pipéridinamine, dichlorhydrate**

*Stade A: Trans-1-{3-[(3,5-dibromobenzyl)oxy]-3,4-dihydro-2H-chromén-4-yl}-N-méthyl-4-pipéridinylcarbamate de tert-butyle*

On ajoute 0,2 g d'hydrure de sodium (à 60% dans l'huile) à 1g du composé obtenu au stade B de l'Exemple 9 et 0,42 ml d'iodure de méthyle dans 10 ml de tétrahydrofurane, en maintenant la température du milieu réactionnel à 0°C. Après 15 minutes à cette température, on laisse sous agitation pendant 48h à température ambiante, dilue à l'eau et extrait par de l'acétate d'éthyle. Après traitement habituel on isole le produit attendu.

*Stade B: Trans-1-{3-[(3,5-dibromobenzyl)oxy]-3,4-dihydro-2H-chromén-4-yl}-N-méthyl-4-pipéridinamine, dichlorhydrate*

Le produit attendu est obtenu selon le procédé décrit au stade C de l'Exemple 1, à partir du composé obtenu au stade précédent.
Point de fusion : 192-195°C.

**EXEMPLE 53 : Trans-1-{3-[(3,5-dibromobenzyl)oxy)-3,4-dihydro-2*H*-chromén-4-yl}-N,N-diméthyl-4-pipéridinamine, dichlorhydrate**

*Stade A: Trans-1-{3-[(3,5-dibromobenryl)oxy]-3,4-dihydro-2H-chromén-4-yl)-N-méthyl-4-pipéridinamine*

Le produit attendu est obtenu par retour base du composé de l'Exemple 52.

*Stade B: Trans-1-{3-[(3,5-dibromobenzyl)oxy]-3,4-dihydro-2H-chromén-4 yl}-N,N-diméthyl-4-pipéridinamine*

Le produit attendu est obtenu selon le procédé décrit au stade A de l'Exemple 52, à partir du composé obtenu au stade précédent.

*Stade C: Trans-1-{3-[(3,5-dibromobenzyl)oxy]-3,4-dihydro-2H-chromén-4-yl)-N,N-diméthyl-4-pipéridinamine, dichlorhydrate*

Le produit attendu est obtenu par salification à l'aide d'acide chlorhydrique du composé obtenu au stade précédent.
Point de fusion : 187-190°C.

**<u>EXEMPLE 54</u> : Trans-1-{3-[(3,5-diméthoxybenzyl)oxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3,5-diméthoxybenzyle.
*<u>Point de fusion</u>* : 108-115°C.

**<u>EXEMPLE 55</u> : Trans-1-{3-benzyloxy-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de benzyle.
*<u>Point de fusion</u>* : 66-80°C.

**<u>EXEMPLE 56</u> : Trans-1-{3-[(3-fluorobenzyl)oxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3-fluorobenzyle.
*<u>Point de fusion</u>* : 180-184°C.

**<u>EXEMPLE 57</u> : Trans-1-{3-[(3-chlorobenzyl)oxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3-chlorobenzyle.
*<u>Point de fusion</u>* : 97-107°C.

**<u>EXEMPLE 58</u> : Trans-1-{3-[(3,4-dichlorobenzyl)oxy]-3,4-dihydro-2*H*-chromén-4-yl}pipérazine, dichlorhydrate**

Le produit attendu est obtenu selon le procédé de l'Exemple 4, en remplaçant au stade D le bromure de 3,5-dibromobenzyle par le bromure de 3,4-dichlorobenzyle.
*<u>Point de fusion</u> :* 114-121°C.

**<u>EXEMPLE 59</u> : Trans-1-{2-[(3-chloro-5-fluorobenzyl)oxy]-2,3-dihydro-1*H*-indén-1-yl}pipérazine, diméthanesulfonate**

*<u>Stade A</u>: Trans-1-{2-[(3-chloro-5-fluorobenzyl)oxy]-2,3-dihydro-1H-indén-1-yl}pipérazine, dichlorhydrate*

Le produit attendu est obtenu selon le procédé décrit aux stades B et C de l'Exemple 1, en remplaçant au stade B le bromure de 3,5-dibromobenzyle par le bromure de 3-chloro-5-fluorobenzyle.

*<u>Stade B</u>: Trans-1-{2-[(3-chloro-5-fluorobenzyl)oxy]-2,3-dihydro-1H-indén-1-yl}pipérazine, diméthanesulfonate*

Le produit attendu est obtenu par retour base du composé obtenu au stade précédent, suivi d'une salification à l'aide d'acide méthanesulfonique.
*<u>Point de fusion</u> :* 175-182°C.

**<u>EXEMPLE 60</u> : Trans-1-{3-[(3-(trifluorométhyl)-benzyl)oxy]-3,4-dihydro-2*H* chromén-4-yl}pipérazine, diméthanesulfonate**

*<u>Stade A</u>: Trans-1-{3-[(3-(trifluorométhyl)-benzyl)oxy]-3,4-dihydro-2H-chromén-4-yl}pipérazine, dichlorhydrate*

Le produit attendu est obtenu selon le procédé de l'Exemple 4, en remplaçant au stade D le bromure de 3,5-dibromobenzyle par le bromure de 3-(trifluorométhyl)-benzyle.

*<u>Stade B:</u> Trans-1-{3-[(3-(trifluorométhyl)-benzyl)oxy]-3,4-dihydro-2H-chromén-4-yl}pipérazine, diméthanesulfonate*

Le produit attendu est obtenu par retour base du composé obtenu au stade précédent, suivi d'une salification à l'aide d'acide méthanesulfonique.
*<u>Point de fusion</u>* : 123-127°C.

**EXEMPLE 61 : Trans-1-{3-[(3-cyanobenzyl)oxy]-3,4-dihydro-2*H*-chromén-4-yl}pipérazine, diméthanesulfonate**

*Stade A: Trans-1-{3-[(3-cyanobenzyl)oxy]-3,4-dihydro-2H-chromén-4yl}pipérazine, dichlorhydrate*

Le produit attendu est obtenu selon le procédé de l'Exemple 4, en remplaçant au stade D le bromure de 3,5-dibromobenzyle par le bromure de 3-cyanobenzyle.

*Stade B: Trans-1-{3-[(3-cyanobenzyl)oxy]-3,4-dihydro-2H-chromén-4-yl}pipérazine, diméthanesulfonate*

Le produit attendu est obtenu par retour base du composé obtenu au stade précédent, suivi d'une salification à l'aide d'acide méthanesulfonique.
*Point de fusion* : 118-121°C.

**EXEMPLE 62 : Isomère (+) de la trans-1-{3-(3-chloro-5-fluorobenzyloxy)-3,4-dihydro-2H-chromén-4-yl}pipérazine, dibenzoyltartrate (+)**

*Stade A : Isomère* (+) *du trans-4-{3-[(3-chloro-5-fluorobenzyl)oxy]-3,4-dihydro-2H-chromén-4-yl}pipérazine-1-carboxylate de tert-butyle*

Le composé attendu est obtenu par séparation par chromatographie HPLC préparative chirale du mélange racémique obtenu au stade A de l'Exemple 47.

*Stade B : Isomère* (+) *de la trans-1-{3-(3-chloro-5-fluorobenzyloxy)-3,4-dihydro-2H-chromén-4-yl}pipérazine, dichlorhydrate*

Le produit attendu est obtenu selon le procédé décrit au stade C de l'Exemple 1, à partir du composé obtenu au stade précédent.

*Stade C : Isomère* (+) *de la trans-1-{3-(3-chloro-5-fluorobenzyloxy)-3,4-dihydro-2H chromén-4-yl}pipérazine, dibenzoyltartrate* (+)

Le produit attendu est obtenu par retour base du composé obtenu au stade précédent, suivi d'une salification par l'acide dibenzoyltatrique (+).
*Point de fusion* : 100-107°C.

**EXEMPLE 63 : Isomère (-) de la trans-1-{3-(3-chloro-5-fluorobenzyloxy)-3,4-dihydro-2H-chromén-4-yl}pipérazine, dibenzoyltartrate (-)**

*Stade A : Isomère (-) du trans-4-{3-[(3-chloro-5-fluorobenzyl)oxy]-3,4-dihydro-2H-chromén-4-yl} pipérazine-1- carboxylate de tert-butyle*

Le composé attendu est le deuxième des énantiomères séparés au stade A de l'Exemple 62.

*Stade B : Isomère (-) de la trans-1-{3-(3-chloro-5-fluorobenzyloxy)-3,4-dihydro-2H chromén-4-yl}pipérazine, dichlorhydrate*

Le produit attendu est obtenu selon le procédé décrit au stade C de l'Exemple 1, à partir du composé obtenu au stade précédent.

*Stade C : Isomère (-) de la trans-1-{3-(3-chloro-5-fluorobenzyloxy)-3,4-dihydro-2H-chromén-4-yl}pipérazine, dibenzoyltartrate (-)*

Le produit attendu est obtenu par retour base du composé obtenu au stade précédent, suivi d'une salification par l'acide dibenzoyltatrique (-).
*Point de fusion :* 100-107°C.

**EXEMPLE 64 : Trans-1-{3-[(3,5-difluorobenzyl)oxy]-3,4-dihydro-2*H*-chromén-4-yl}pipérazine, diméthanesulfonate**

*StadeA: Trans-1-{3-[(3,5-difluorobenzyl)oxy]-3,4-dihydro-2H-chromén-4 yl}pipérazine, dichlorhydrate*

Le produit attendu est obtenu selon le procédé de l'Exemple 4, en remplaçant au stade D le bromure de 3,5-dibromobenzyle par le bromure de 3,5-difluorobenzyle.

*Stade B: Trans-1-{3-[(3-difluorobenzyl)oxy]-3,4-dihydro-2H-chromén-4yl}pipérazine, diméthanesulfonate*

Le produit attendu est obtenu par retour base du composé obtenu au stade précédent, suivi d'une salification à l'aide d'acide méthanesulfonique.
*Point de fusion :* 178-182°C.

**EXEMPLE 65 : Trans-4-{3-[(3,5-dibromobenzyl)oxy]-3,4-dihydro-2*H*-chromén-4-yl}pipéridine, chlorhydrate**

Le produit attendu est obtenu selon le procédé de l'Exemple 50, en remplaçant au stade A la 1-tétralone par la 2,3-dihydro-4H-chromén-4-one.
*Point de fusion* : 148-167°C.

**EXEMPLE 66 : Trans-1-{3-[(3-(trifluorométhoxy)-benzyl)oxy]-3,4-dihydro-2*H*-chromén-4-yl}pipérazine, diméthanesulfonate**

*Stade A: Trans-1-{3-[(3-(trifluorométhoxy)-benzyl)oxy]-3,4-dihydro-2H-chromén-4-yl}pipérazine, dichlorhydrate*

Le produit attendu est obtenu selon le procédé de l'Exemple 4, en remplaçant au stade D le bromure de 3,5-dibromobenzyle par le bromure de 3-(trifluorométhoxy)-benzyle.

*Stade B: Trans-1-[3-[(3-(trifluorométhoxy)-benzyl)oxy]-3,4-dihydro-2H-chromén-4-yl}pipérazine, diméthanesulfonate*

Le produit attendu est obtenu par retour base du composé obtenu au stade précédent, suivi d'une salification à l'aide d'acide méthanesulfonique.
*Point de fusion :* 132-135°C.

**ETUDE PHARMACOLOGIQUE DES DERIVES DE L'INVENTION**

**EXEMPLE 67 : Détermination de l'affinité pour les sites de recapture de la sérotonine chez le rat.**

L'affinité des composés pour le site de recapture de la sérotonine (5-HTT) est évaluée par des expériences de compétition avec le [$^3$H]-citalopram sur membranes de cortex frontal de rat. Les cortex sont homogénéisés au Polytron dans 40 volumes (poids/volume) de tampon d'incubation Tris-HCl (50 mM, pH 7,4) froid contenant 120 mM de NaCl et 5 mM de KCl puis centrifugés une première fois. Le culot est re-suspendu dans le même tampon et incubé 10 min à 37°C puis re-centrifugé. Les membranes sont lavées encore deux fois puis le culot est re-suspendu dans un volume approprié de tampon d'incubation. Les membranes sont ensuite incubées pendant 2 heures à 25°C avec le composé étudié en présence de 0.7 nM de [$^3$H]-citalopram. La liaison non spécifique est déterminée avec 10 μM de fluoxétine. A la fin de la période d'incubation, les échantillons sont filtrés au travers de filtres de type Unifilter GF/B pré-traités au PEI (0.05%) et lavés plusieurs fois avec le tampon d'incubation. La radioactivité retenue sur les filtres est comptée après adjonction de liquide scintillant à l'aide d'un compteur à scintillation. Les isothermes obtenus sont analysés par régression non linéaire afin de déterminer des valeurs d'$IC_{50}$ qui sont converties en $K_i$ par l'équation de Cheng-Prussof :

$$K_i = IC_{50}/(1 + L/k_D)$$

dans laquelle L représente la concentration en radioligand et le $k_D$ est la constante de dissociation du [$^3$H]-citalopram sur le site de recapture de la sérotonine (0.7 nM). Les résultats sont exprimés en $pK_i$ = -log $K_i$.
Les résultats obtenus pour les composés représentatifs de l'invention sont rassemblés dans le tableau suivant :

| Composé | $pK_1$ r5-HTT |
|---|---|
| Exemple 1 | 8,35 |
| Exemple 4 | 7,10 |
| Exemple 21 | 7,91 |
| Exemple 36 | 8,97 |
| Exemple 38 | 8,73 |
| Exemple 42 | 7,71 |
| Exemple 44 | 7,31 |
| Exemple 47 | 7,45 |

**EXEMPLE 68 : Binding $hNK_1$.**

L'affinité des composés de l'invention a été déterminée par des expériences de compétition en présence de [$^3$H]-Substance P (Sar-9, MetO2-11,2-propyl-3,4-3H). Les cellules de lymphoblastes humains IM9 exprimant les récepteurs $NK_1$ de manière endogène sont centrifugées et reprises dans le tampon d'incubation contenant 50mM de TRIS, 150mM de NaCl, 4mM de $CaCl_2$, des inhibiteurs de protéases au 1/100$^e$ (Cocktail SIGMA P8340) et 0,2% de BSA. Le volume de tampon d'incubation est déterminé de manière à obtenir une concentration de $5.10^6$ cellules / ml. Cette préparation cellulaire est ensuite incubée avec 1.5 nM de [$^3$H]-Substance P et le composé étudié, pendant 90 min à température ambiante. La liaison non spécifique est déterminée en présence de 1 μM de GR 205171.
A la fin de la période d'incubation, les échantillons sont filtrés au travers de filtres de type Unifilter GF/B pré-traités au PEI (0.1 %) et lavés plusieurs fois avec le tampon de filtration (50mM de TRIS, 150mM de NaCl, 4mM de $CaCl_2$). La radioactivité retenue sur les filtres est mesurée par comptage après adjonction, sur les filtres, de liquide scintillant. Les comptages sont analysés par régression non-linéaire permettant de tracer les isothermes et de déterminer les valeurs d'$IC_{50}$ qui sont ensuite converties en constante d'inhibition ($K_i$) par l'intermédiaire de l'équation de Cheng-Prusoff :

$$K_i = IC_{50} / (1 + L / K_D)$$

dans laquelle L est la concentration de [$^3$H]-Substance P et $K_D$ est la constante de dissociation de [$^3$H]-Substance P pour les récepteurs $NK_1$ humains (0,53 nM). Les résultats sont exprimés en $pK_i$(-log $K_i$).
Les résultats obtenus pour les composés représentatifs de l'invention sont rassemblés dans le tableau suivant :

| Composé | $pK_1$ $NK_1$ |
|---|---|
| Exemple 1 | 6,84 |
| Exemple 4 | 8,25 |
| Exemple 7 | 8,14 |
| Exemple 8 | 7,54 |
| Exemple 9 | 7,96 |
| Exemple 13 | 6,60 |
| Exemple 15 | 7,48 |
| Exemple 19 | 7,40 |
| Exemple 40 | 6,85 |

**EXEMPLE 69 : Composition pharmaceutique.**

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |

(suite)

| Formule de préparation pour 1000 comprimés dosés à 10 mg : | |
|---|---|
| Stéarate de magnésium | 3 g |
| Talc | 3 g |

## Revendications

**1.** Composé de formule (I) :

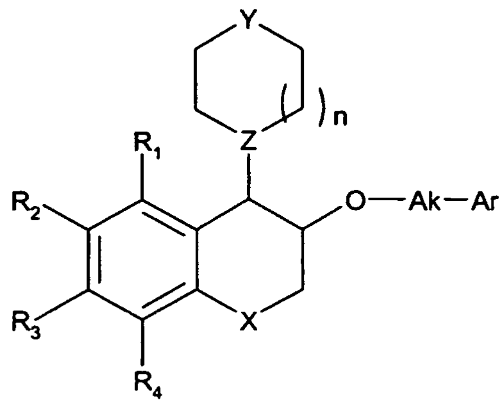

(I)

dans laquelle :

- $R_1$, $R_2$, $R_3$ et $R_4$, identiques ou différents, représentent chacun un atome ou groupement choisi parmi H, halogène, alkyle $C_1$-$C_6$ linéaire ou ramifié, alkoxy $C_1$-$C_6$ linéaire ou ramifié, phényle et cyano,
- X représente une liaison, un atome d'oxygène ou un groupement choisi parmi $-(CH_2)_m-$, $-OCH_2-$ et $-NR_5-$, m représente 1 ou 2,
$R_5$ représente un atome d'hydrogène ou un groupement choisi parmi alkyle $C_1$-$C_6$ linéaire ou ramifié, $COR_6$ et $CO_2R_6$,
$R_6$ représente un groupement alkyle $C_1$-$C_6$ linéaire ou ramifié,
- Y représente un atome d'oxygène ou un groupement choisi parmi $NR_7$ et $CHR_8$,
$R_7$ représente un atome d'hydrogène ou un groupement choisi parmi $COR_9$ et alkyle $C_1$-$C_6$ linéaire ou ramifié éventuellement substitué par un groupement 5-oxo-4,5-dihydro-1*H*-1,2,4-triazol-3-yle ou 2,3-dihydro-1,4-benzodioxin-2-yle,
$R_9$ représente un groupement choisi parmi alkyle $C_1$-$C_6$ linéaire ou ramifié, aryle et hétéroaryle,
$R_8$ représente un atome d'hydrogène ou un groupement amino éventuellement substitué par un ou deux groupements alkyle $C_1$-$C_6$ linéaire ou ramifié,
- Z représente un atome d'azote ou un groupement CH,
- n représente 1 ou 2,
- Ak représente une chaîne alkylène $C_1$-$C_6$ linéaire ou ramifiée,
- Ar représente un groupement aryle ou hétéroaryle,

ses isomères optiques, ainsi que leurs sels d'addition à un acide pharmaceutiquement acceptable,
étant entendu que
par groupement aryle, on entend phényle, biphénylyle ou naphtyle, chacun de ces groupements étant éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano, trihalogénoalkyle $C_1$-$C_6$ linéaire ou ramifié et trihalogénoalkoxy $C_1$-$C_6$ linéaire ou ramifié,
et par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano et trihalogénoalkyle $C_1$-$C_6$ linéaire ou ramifié.

**2.** Composé de formule (I) selon la revendication 1, pour lequel Y représente NH.

**3.** Composé de formule (I) selon l'une quelconque des revendications 1 ou 2, pour lequel Z représente un atome d'azote.

**4.** Composé de formule (I) selon l'une quelconque des revendications 1 à 3, pour lequel n représente 1.

**5.** Composé de formule (I) selon l'une quelconque des revendications 1 à 4, pour lequel Ar représente un groupement aryle.

**6.** Composé de formule (I) selon l'une quelconque des revendications 1 à 5, pour lequel X représente une liaison, un atome d'oxygène ou un groupement-$OCH_2$- ou-$(CH_2)_m$- où m représente 1 ou 2.

**7.** Composé de formule (I) selon la revendication 1, choisi parmi :

- la trans-1-{2-[(3,5-dibromobenzyl)oxy]-2,3-dihydro-1*H*-indén-1-yl}pipérazine, ses énantiomères, ainsi que ses sels d'addition à un acide pharmaceutiquement acceptable,
- la trans-1-{3-[(3,5-dibromobenzyl)oxy]-3,4-dihydro-2*H*-chromén-4-yl}pipérazine, ses énantiomères, ainsi que ses sels d'addition à un acide pharmaceutiquement acceptable,
- la trans-1-{6-[(3,5-dibromobenzyl)oxy]-6,7,8,9-tétrahydro-5*H*-benzo[7]annulén-5-yl}pipérazine, ses énantiomères, ainsi que ses sels d'addition à un acide pharmaceutiquement acceptable,
- la trans-1-{2-[(3,5-dibromobenzyl)oxy]-1,2,3,4-tétrahydro-naphthalén-1-yl}pipérazine, ses énantiomères, ainsi que ses sels d'addition à un acide pharmaceutiquement acceptable,
- le trans-1-{2-[(3,5-dibromobenzyl)oxy]-1,2,3,4-tétrahydro-naphthalén-1-yl}-1,4-diazépane, ses énantiomères, ainsi que ses sels d'addition à un acide pharmaceutiquement acceptable,
- la 1-{(1*S*,2*R*)-2-[(3,5-dibromobenzyl)oxy]-2,3-dihydro-1*H*-indén-1-yl}pipérazine, ses isomères optiques, ainsi que ses sels d'addition à un acide pharmaceutiquement acceptable,
- la 1-[(1S,2R)-2-[(3,5-Difluorobenzyl)-oxy]-2,3-dihydro-1*H*-indén-1-yl]-pipérazine, ses isomères optiques, ainsi que ses sels d'addition à un acide pharmaceutiquement acceptable,
- la 1-[(1S,2R)-2-[(3,5-Diméthylbenzyl)-oxy]-2,3-dihydro-1*H*-indén-1-yl]-pipérazine, ses isomères optiques, ainsi que ses sels d'addition à un acide pharmaceutiquement acceptable,
- la trans-1-{3-[(3,5-dichlorobenzyl)oxy]-3,4-dihydro-2*H*-chromén-4-yl}pipérazine, ses énantiomères, ainsi que ses sels d'addition à un acide pharmaceutiquement acceptable,
- la trans-1-{3-[3-fluoro-5-(trifluorométhyl)benzyloxy]-3,4-dihydro-2*H*-chromén-4-yl}pipérazine, ses énantiomères, ainsi que ses sels d'addition à un acide pharmaceutiquement acceptable,
- et la trans-1-{3-(3-chloro-5-fluorobenzyloxy)-3,4-dihydro-2H-chromén-4-yl}pipérazine, ses énantiomères, ainsi que ses sels d'addition à un acide pharmaceutiquement acceptable.

**8.** Procédé de préparation des composés de formule (I) selon la revendication 1 à partir du composé de formule (Va), de configuration relative trans :

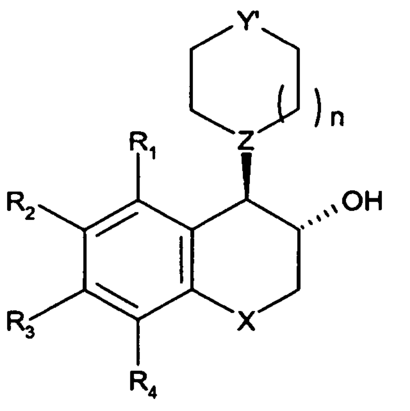

(Va)

dans laquelle $R_1$, $R_2$, $R_3$, $R_4$, X, n et Z sont tels que définis précédemment, et Y' représente un atome d'oxygène ou un groupement choisi parmi $NP_1$ et $CHR'_8$, où $R'_8$ représente un atome d'hydrogène ou un groupement $NHP_1$, et $P_1$ représente un groupement protecteur de la fonction amine,

- composé de formule (Va) qui est mis en réaction, lorsque l'on souhaite accéder aux composés de formule (I) de configuration relative trans, avec un composé de formule (VI):

G-Ak-Ar (VI)

dans laquelle Ak et Ar sont tels que définis dans la formule (I), et G représente un groupement partant tel que, par exemple, un atome d'halogène ou un groupement p-toluènesulfonate, trifluorométhanesulfonate ou méthanesulfonate, pour conduire au composé de formule (VIIa), de configuration relative trans :

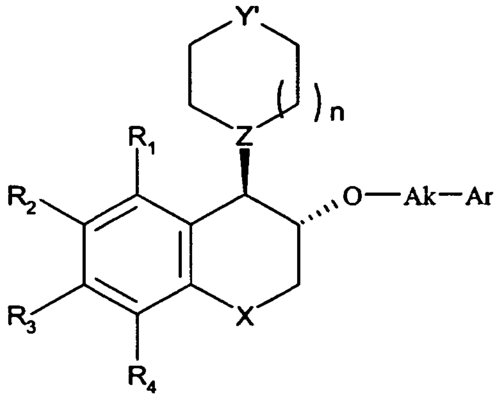

(VIIa)

dans laquelle $R_1$, $R_2$, $R_3$, $R_4$, X, n, Y', Z, Ak et Ar sont tels que définis précédemment,
que l'on déprotège, lorsque Y' comporte un groupement protecteur $P_1$ tel que défini précédemment, puis que l'on alkyle, lorsqu'on souhaite accéder à des composés pour lesquels Y représente un groupement $NR_7$ dans lequel $R_7$ est autre qu'un atome d'hydrogène,
pour conduire aux composés de formule (Ia), cas particulier des composés de formule (I) qui sont de configuration relative trans :

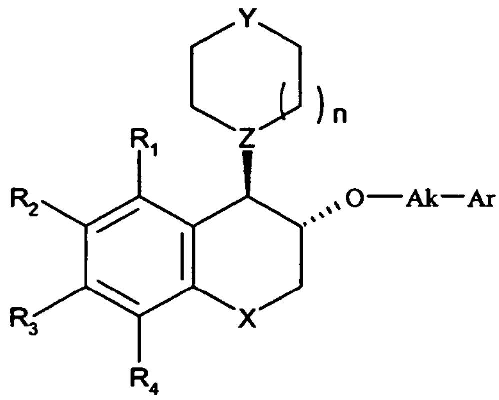

(Ia)

dans laquelle $R_1$, $R_2$, $R_3$, $R_4$, X, n, Y, Z, Ak et Ar sont tels que définis dans la formule (I),
- ou bien composé de formule (Va) qui est oxydé, lorsque l'on souhaite accéder aux composés de formule (I) de configuration relative cis,

pour conduire au composé racémique de formule (VIII) :

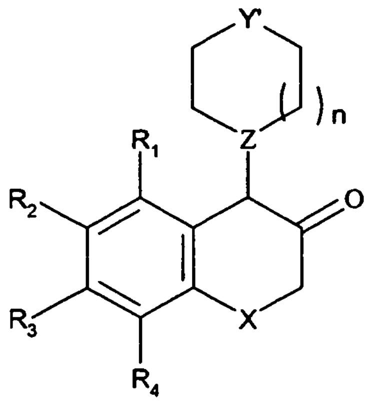

(VIII)

dans laquelle $R_1$, $R_2$, $R_3$, $R_4$, X, Z, n et Y' sont tels que définis précédemment, qui est réduit en alcool correspondant, dont les diastéréoisomères sont séparés et l'isomère de formule (Vb), de configuration relative cis, isolé :

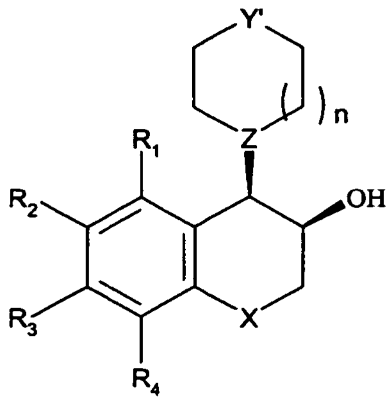

(Vb)

dans laquelle $R_1$, $R_2$, $R_3$, $R_4$, X, Y', Z et n sont tels que définis précédemment, que l'on met en réaction avec un composé de formule (VI) tel que défini précédemment, pour conduire au composé de formule (VIIb), de configuration relative cis :

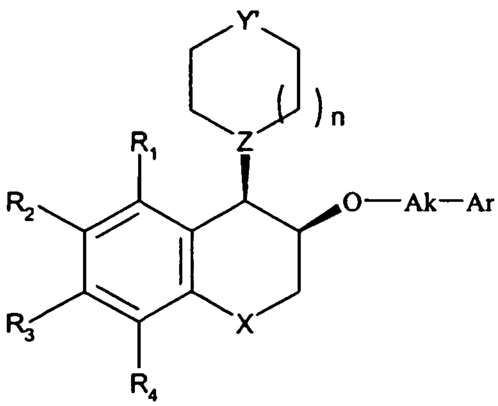

(VIIb)

dans laquelle $R_1$, $R_2$, $R_3$, $R_4$, X, n, Y', Z, Ak et Ar sont tels que définis précédemment, que l'on déprotège, lorsque Y' comporte un groupement protecteur $P_1$ tel que défini précédemment, puis que l'on alkyle, lorsqu'on souhaite accéder à des composés pour lesquels Y représente un groupement $NR_7$ dans lequel $R_7$ est autre qu'un atome d'hydrogène, pour conduire aux composés de formule (Ib), cas particulier des composés de formule (I) qui sont de configuration

relative cis :

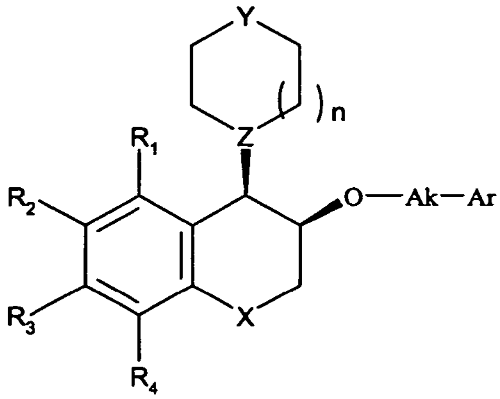

(Ib)

dans laquelle $R_1$, $R_2$, $R_3$, $R_4$, X, Y, Z, n, Ak et Ar sont tels que définis dans la formule (I), composés de formule (Ia) et (Ib) que l'on purifie, lorsqu'on le souhaite, selon une technique classique de purification, dont on sépare, lorsqu'on le souhaite, les isomères optiques, et que l'on transforme, lorsqu'on le souhaite, en leurs sels d'addition à un acide pharmaceutiquement acceptable.

**9.** Composition pharmaceutique contenant comme principe actif un composé selon l'une quelconque des revendications 1 à 7, en combinaison avec un ou plusieurs véhicules inertes, non toxiques et pharmaceutiquement acceptables.

**10.** Utilisation des composés de formule (I) selon l'une quelconque des revendications 1 à 7 pour la fabrication de médicaments utiles en tant qu'inhibiteurs de recapture de sérotonine.

**11.** Utilisation des composés de formule (I) selon l'une quelconque des revendications 1 à 7 pour la fabrication de médicaments utiles en tant qu'inhibiteurs de recapture de sérotonine et antagonistes $NK_1$.

**12.** Utilisation des composés de formule (I) selon l'une quelconque des revendications 1 à 7 pour la fabrication de médicaments utiles pour le traitement des états dépressifs, des états anxieux, des troubles impulsifs, des comportements agressifs, de l'abus de drogue, de l'obésité et des troubles de l'appétit, de la douleur et l'inflammation, des démences, des états psychotiques, des perturbations des rythmes chronobiologiques, de la nausée ou des troubles gastro-intestinaux.

## Claims

**1.** Compound of formula (I) :

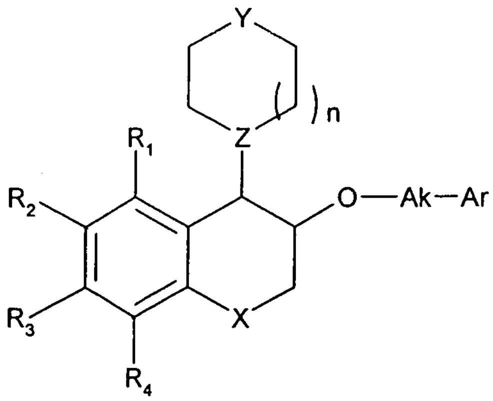

(I),

wherein :

- $R_1$, $R_2$, $R_3$ and $R_4$, which may be the same or different, each represent an atom or group selected from H, halogen, linear or branched $C_1$-$C_6$alkyl, linear or branched $C_1$-$C_6$alkoxy, phenyl and cyano,
- X represents a bond, an oxygen atom or a group selected from $-(CH_2)_m-$, $-OCH_2-$ and $-NR_5-$,
m represents 1 or 2,
$R_5$ represents a hydrogen atom or a group selected from linear or branched $C_1$-$C_6$alkyl, $COR_6$ and $CO_2R_6$,
$R_6$ represents a linear or branched $C_1$-$C_6$alkyl group,
- Y represents an oxygen atom or a group selected from $NR_7$ and $CHR_8$,
$R_7$ represents a hydrogen atom or a group selected from $COR_9$ and linear or branched $C_1$-$C_6$alkyl, the alkyl group being optionally substituted by a 5-oxo-4,5-dihydro-1*H*-1,2,4-triazol-3-yl or 2,3-dihydro-1,4-benzodioxin-2-yl group,
$R_9$ represents a group selected from linear or branched $C_1$-$C_6$alkyl, aryl and heteroaryl,
$R_8$ represents a hydrogen atom or an amino group optionally substituted by one or two linear or branched $C_1$-$C_6$alkyl groups,
- Z represents a nitrogen atom or a CH group,
- n represents 1 or 2,
- Ak represents a linear or branched $C_1$-$C_6$alkylene chain,
- Ar represents an aryl or heteroaryl group,

its optical isomers, and also addition salts thereof with a pharmaceutically acceptable acid,
it being understood that
an aryl group means phenyl, biphenylyl or naphthyl, each of those groups optionally being substituted by one or more identical or different groups selected from halogen, linear or branched $C_1$-$C_6$alkyl, linear or branched $C_1$-$C_6$alkoxy, hydroxy, cyano, linear or branched $C_1$-$C_6$trihaloalkyl and linear or branched $C_1$-$C_6$trihaloalkoxy,
and a heteroaryl group means an aromatic mono- or bi-cyclic 5- to 12-membered group containing one, two or three hetero atoms selected from oxygen, nitrogen and sulphur, it being understood that the heteroaryl group may optionally be substituted by one or more identical or different groups selected from halogen, linear or branched $C_1$-$C_6$alkyl, linear or branched $C_1$-$C_6$alkoxy, hydroxy, cyano and linear or branched $C_1$-$C_6$trihaloalkyl.

**2.** Compound of formula (I) according to claim 1, wherein Y represents NH.

**3.** Compound of formula (I) according to either claim 1 or claim 2, wherein Z represents a nitrogen atom.

**4.** Compound of formula (I) according to any one of claims 1 to 3, wherein n represents 1.

**5.** Compound of formula (I) according to any one of claims 1 to 4, wherein Ar represents an aryl group.

**6.** Compound of formula (I) according to any one of claims 1 to 5, wherein X represents a bond, an oxygen atom or an $-OCH_2-$ or $-(CH_2)_m-$ group wherein m represents 1 or 2.

**7.** Compound of formula (I) according to claim 1 selected from :

- *trans*-1-{2-[(3,5-dibromobenzyl)oxy]-2,3-dihydro-1*H*-inden-1-yl}piperazine, its enantiomers, and also addition salts thereof with a pharmaceutically acceptable acid,
- *trans*-1-{3-[(3,5-dibromobenzyl)oxy]-3,4-dihydro-2*H*-chromen-4-yl}piperazine, its enantiomers, and also addition salts thereof with a pharmaceutically acceptable acid,
- *trans*-1-{6-[(3,5-dibromobenzyl)oxy]-6,7,8,9-tetrahydro-5*H*-benzo[7]annulen-5-yl}piperazine, its enantiomers, and also addition salts thereof with a pharmaceutically acceptable acid,
- *trans*-1-{2-[(3,5-dibromobenzyl)oxy]-1,2,3,4-tetrahydronaphth-1-yl}piperazine, its enantiomers, and also addition salts thereof with a pharmaceutically acceptable acid,
- *trans*-1-{2-[(3,5-dibromobenzyl)oxy]-1,2,3,4-tetrahydronaphth-1-yl}-1,4-diazepane, its enantiomers, and also addition salts thereof with a pharmaceutically acceptable acid,
- 1-{(1*S*,2*R*)-2-[(3,5-dibromobenzyl)oxy]-2,3-dihydro-1*H*-inden-1-yl}piperazine, its enantiomers, and also addition salts thereof with a pharmaceutically acceptable acid,
- 1-[(1S,2R)-2-[(3,5-difluorobenzyl)oxy]-2,3-dihydro-1*H*-inden-1-yl]piperazine, its enantiomers, and also addition salts thereof with a pharmaceutically acceptable acid,
- 1-[(1S,2R)-2-[(3,5-dimethylbenzyl)oxy]-2,3-dihydro-1*H*-inden-1-yl]piperazine, its enantiomers, and also addition salts thereof with a pharmaceutically acceptable acid,
- *trans*-1-{3-[(3,5-dichlorobenzyl)oxy]-3,4-dihydro-2*H*-chromen-4-yl}piperazine, its enantiomers, and also addition salts thereof with a pharmaceutically acceptable acid,
- trans-1-{3-[3-fluoro-5-(trifluoromethyl)benzyloxy]-3,4-dihydro-2*H*-chromen-4-yl}piperazine, its enantiomers, and also addition salts thereof with a pharmaceutically acceptable acid,
- and *trans*-1-{3-(3-chloro-5-fluorobenzyloxy)-3,4-dihydro-2H-chromen-4-yl}piperazine, its enantiomers, and also addition salts thereof with a pharmaceutically acceptable acid.

**8.** Process for the preparation of compounds of formula (I) according to claim 1, starting from the compound of formula (Va), of relative configuration trans :

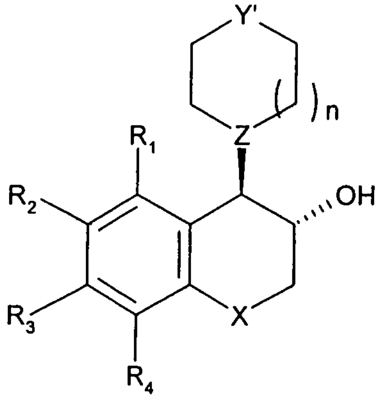

(Va),

wherein $R_1$, $R_2$, $R_3$, $R_4$, X, n and Z are as defined hereinbefore, and Y' represents an oxygen atom or a group selected from $NP_1$ and $CHR'_8$, wherein $R'_8$ represents a hydrogen atom or a group $NHP_1$, and $P_1$ represents a protecting group for the amine function,

- which compound of formula (Va) is reacted, when it is desired to obtain compounds of formula (I) of relative configuration *trans,* with a compound of formula (VI) :

G-Ak-Ar (VI),

wherein Ak and Ar are as defined for formula (I), and G represents a leaving group such as, for example, a halogen atom or a p-toluenesulphonate, trifluoromethanesulphonate or methanesulphonate group,
to yield the compound of formula (VIIa), of relative configuration *trans :*

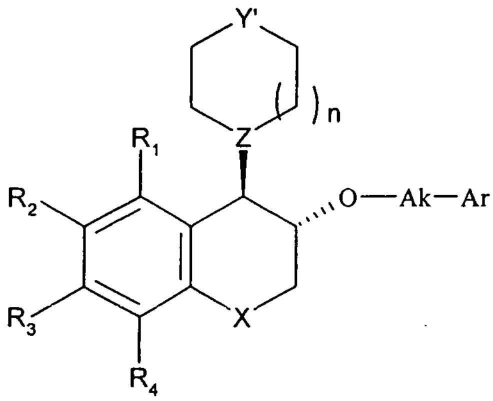

(VIIa),

wherein $R_1$, $R_2$, $R_3$, $R_4$, X, n, Y', Z, Ak and Ar are as defined hereinbefore,
which is deprotected when Y' contains a protecting group $P_1$ as defined hereinbefore and is then alkylated, when it is desired to obtain compounds wherein Y represents a group $NR_7$ wherein $R_7$ is other than a hydrogen atom,

to yield compounds of formula (Ia), a particular case of the compounds of formula (I), which are of relative configuration *trans :*

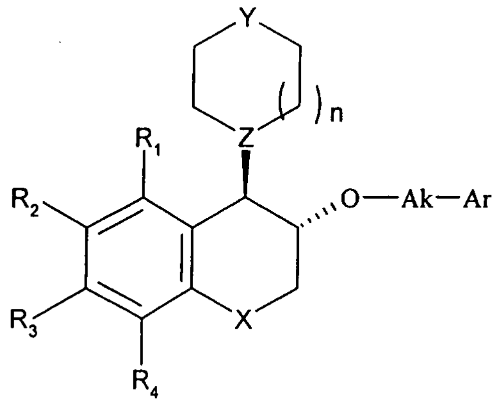

(Ia),

wherein $R_1$, $R_2$, $R_3$, $R_4$, X, n, Y, Z, Ak and Ar are as defined for formula (I),
- or which compound of formula (Va) is oxidised, when it is desired to obtain compounds of formula (I) of relative configuration *cis,*

to yield the racemic compound of formula (VIII) :

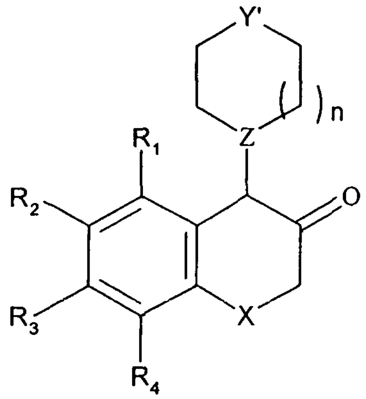

(VIII),

wherein $R_1$, $R_2$, $R_3$, $R_4$, X, Z, n and Y' are as defined hereinbefore,
which is reduced to the corresponding alcohol, the diastereoisomers of which are separated, and the isomer of formula (Vb), of relative configuration *cis,* is isolated :

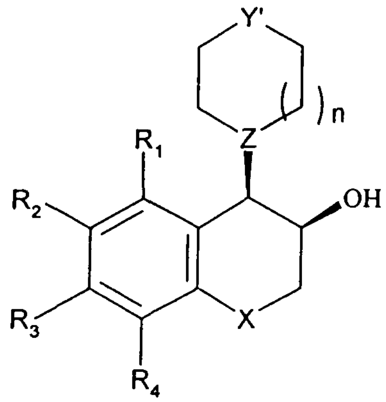

(Vb),

wherein $R_1$, $R_2$, $R_3$, $R_4$, X, Y', Z and n are as defined hereinbefore,
which is reacted with a compound of formula (VI) as defined hereinbefore to yield the compound of formula (VIIb), of relative configuration *cis :*

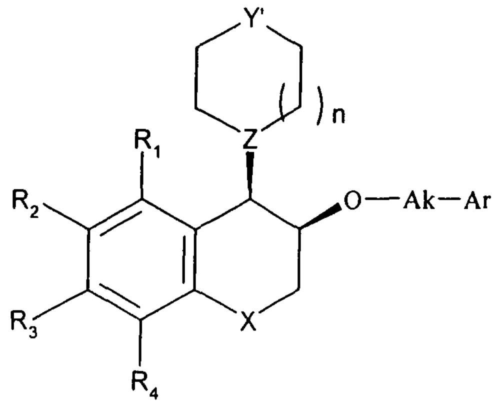

(VIIb),

wherein $R_1$, $R_2$, $R_3$, $R_4$, X, n, Y', Z, Ak and Ar are as defined hereinbefore, which is deprotected when Y' contains a protecting group $P_1$ as defined hereinbefore and is then alkylated, when it is desired to obtain compounds wherein Y represents a group $NR_7$ wherein $R_7$ is other than a hydrogen atom,
to yield compounds of formula (Ib), a particular case of the compounds of formula (I), which are of relative configuration *cis :*

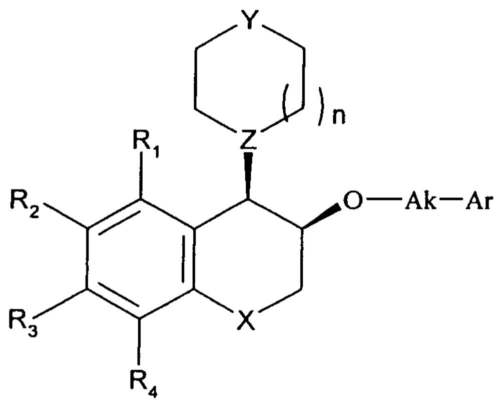

(Ib),

wherein $R_1$, $R_2$, $R_3$, $R_4$, X, Y, Z, n, Ak and Ar are as defined for formula (I),
which compounds of formulae (Ia) and (Ib) are purified, when desired, according to a conventional purification technique, are separated, when desired, into their optical isomers and are converted, when desired, into their addition salts with a pharmaceutically acceptable acid.

**9.** Pharmaceutical composition comprising, as active ingredient, a compound according to any one of claims 1 to 7, in combination with one or more pharmaceutically acceptable, inert, non-toxic carriers.

**10.** Use of compounds of formula (I) according to any one of claims 1 to 7 in the manufacture of medicaments for use as serotonin reuptake inhibitors.

**11.** Use of compounds of formula (I) according to any one of claims 1 to 7 in the manufacture of medicaments for use as serotonin reuptake inhibitors and $NK_1$ antagonists.

**12.** Use of compounds of formula (I) according to any one of claims 1 to 7 in the manufacture of medicaments for use in the treatment of depressive states, anxiety states, impulsive disorders, aggressive behaviours, drug abuse, obesity and appetite disorders, pain and inflammation, dementias, psychotic states, disturbances of chronobiological rhythms, nausea or gastrointestinal disorders.

## Patentansprüche

1. Verbindung der Formel (I):

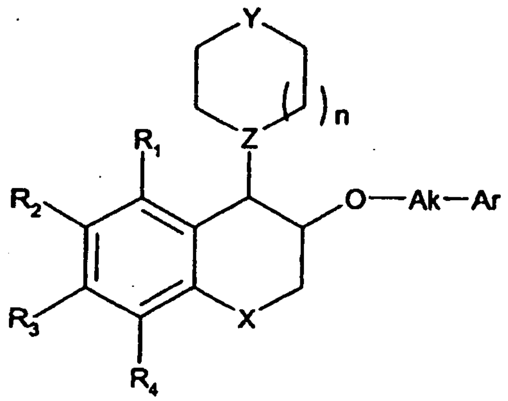

(I)

in der:

- $R_1$, $R_2$, $R_3$ und $R_4$, die gleichartig oder verschieden sind, jeweils ein Atom oder eine Gruppe bedeuten ausgewählt aus H. Halogen, geradkettigem oder verzweigtem $C_1$-$C_6$-Alkyl, geradkettigem oder verzweigtem $C_1$-$C_6$-Alkoxy, Phenyl und Cyano,
- X eine Bindung, ein Sauerstoffatom oder eine Gruppe bedeutet ausgewählt aus: $-(CH_2)_m-$, $-OCH_2-$ und $-NR_5-$, in denen
m 1 oder 2 darstellt,
$R_5$ ein Wasserstoffatom oder eine Gruppe ausgewählt aus geradkettigem oder verzweigtem $C_1$-$C_6$-Alkyl, $COR_6$ und $CO_2R_6$ darstellt und
$R_6$ eine geradkettige oder verzweigte $C_1$-$C_6$-Alkylgruppe darstellt,
- Y ein Sauerstoffatom oder eine Gruppe ausgewählt aus $NR_7$ und $CHR_8$ bedeutet, worin
$R_7$ ein Wasserstoffatom oder eine Gruppe bedeutet ausgewählt aus $COR_9$ und geradkettigem oder verzweigtem $C_1$-$C_6$-Alkyl, welches gegebenenfalls durch eine 5-Oxo-4,5-dihydro-1*H*-1,2,4-triazol-3-yl- oder 2,3-Dihydro-1,4-benzodioxin-2-yl-gruppe substituiert ist,
$R_9$ eine Gruppe bedeutet ausgewählt aus geradkettigem oder verzweigtem $C_1$-$C_6$-Alkyl, Aryl und Heteroaryl,
$R_8$ ein Wasserstoffatom oder eine Aminogruppe bedeutet, die gegebenenfalls durch eine oder zwei geradkettige oder verzweigte $C_1$-$C_6$-Alkylgruppen substituiert ist,
- Z ein Stickstoffatom oder eine Gruppe CH bedeutet,
- n 1 oder 2 bedeutet,
- Ak eine geradkettige oder verzweigte $C_1$-$C_6$-Alkylenkette bedeutet und
- Ar eine Aryl- oder Heteroarylgruppe bedeutet,

ihre optischen Isomere sowie ihre Additionssalze mit einer pharmazeutisch annehmbaren Säure,
wobei es sich versteht, daß man
unter einer Arylgruppe Phenyl, Biphenylyl oder Naphthyl versteht, wobei jede dieser Gruppen gegebenenfalls durch eine oder mehrere gleichartige oder verschiedenartige Gruppen substituiert ist, ausgewählt aus Halogen, geradkettigem oder verzweigtem $C_1$-$C_6$-Alkyl, geradkettigem oder verzweigtem $C_1$-$C_6$-Alkoxy, Hydroxy, Cyano, geradkettigem oder verzweigtem $C_1$-$C_6$-Trihalogenalkyl und geradkettigem oder verzweigtem $C_1$-$C_6$-Trihalogenalkoxy, und
unter einer Heteroarylgruppe eine aromatische, mono- oder bicyclische Gruppe mit 5 bis 12 Kettengliedern versteht, die ein, zwei oder drei Heteroatome enthält ausgewählt aus Sauerstoff, Stickstoff oder Schwefel, wobei es sich versteht, daß die Heteroarylgruppe gegebenenfalls durch eine oder mehrere gleichartige oder verschiedenartige Gruppen substituiert ist, ausgewählt aus Halogen, geradkettigem oder verzweigtem $C_1$-$C_6$-Alkyl, geradkettigem oder verzweigtem $C_1$-$C_6$-Alkoxy, Hydroxy, Cyano und geradkettigem oder verzweigtem $C_1$-$C_6$-Trihalogenalkyl.

2. Verbindung der Formel (I) nach Anspruch 1, worin Y die Gruppe NH bedeutet.

3. Verbindung der Formel (I) nach einem der Ansprüche 1 oder 2, worin Z ein Stickstoffatom bedeutet.

**4.** Verbindung der Formel (I) nach einem der Ansprüche 1 bis 3, worin n den Wert 1 besitzt.

**5.** Verbindung der Formel (I) nach einem der Ansprüche 1 bis 4, worin Ar eine Arylgruppe bedeutet.

**6.** Verbindung der Formel (I) nach einem der Ansprüche 1 bis 5, worin X eine Bindung, ein Sauerstoffatom oder eine Gruppe $-OCH_2-$ oder $-(CH_2)_m-$ , worin m 1 oder 2 bedeutet, darstellt.

**7.** Verbindung der Formel (I) nach Anspruch 1, ausgewählt aus:

- trans-1-{2-[(3,5-Dibrombenzyl)-oxy]-2,3-dihydro-1*H*-inden-1-yl}-piperazin, seine Enantiomeren sowie seine Additionssalze mit einer pharmazeutisch annehmbaren Säure,
- trans-1-{3-[(3,5-Dibrombenzyl)-oxy]-3,4-dihydro-2*H*-chromen-4-yl}-piperazin, seine Enantiomeren sowie seine Additionssalze mit einer pharmazeutisch annehmbaren Säure,
- trans-1-{6-[(3,5-Dibrombenzyl)-oxy]-6,7,8,9-tetrahydro-5*H*-benzo[7]annulen-5-yl}-piperazin, seine Enantiomeren sowie seine Additionssalze mit einer pharmazeutisch annehmbaren Säure,
- trans-1-{2-[(3,5-Dibrombenzyl)-oxy]-1,2,3,4-tetrahydro-naphthalin-1-yl}-piperazin, seine Enantiomeren sowie seine Additionssalze mit einer pharmazeutisch annehmbaren Säure,
- trans-1-{2-[(3,5-Dibrombenzyl)-oxy]-1,2,3,4-tetrahydro-naphthalin-1-yl}-1,4-diazepan, seine Enantiomeren sowie seine Additionssalze mit einer pharmazeutisch annehmbaren Säure,
- 1-{(1S,2R)-2-[(3,5-Dibrombenzyl)-oxy]-2,3-dihydro-1*H*-inden-1-yl}-piperazin, seine optischen Isomeren sowie seine Additionssalze mit einer pharmazeutisch annehmbaren Säure,
- 1-[(1S,2R)-2-[(3,5-Difluorbenzyl)-oxy]-2,3-dihydro-1*H*-inden-1-yl]-piperazin, seine optischen Isomeren sowie seine Additionssalze mit einer pharmazeutisch annehmbaren Säure,
- 1-[(1S,2R)-2-[(3,5-Dimethylbenzyl)-oxy]-2,3-dihydro-1*H*-inden-1-yl]-piperazin, seine optischen Isomeren sowie seine Additionssalze mit einer pharmazeutisch annehmbaren Säure,
- trans-1-{3-[(3,5-Dichlorbenzyl)-oxy]-3,4-dihydro-2*H*-chromen-4-yl}-piperazin, seine Enantiomeren sowie seine Additionssalze mit einer pharmazeutisch annehmbaren Säure,
- trans-1-{3-[3-Fluor-5-(trifluormethyl)-benzyloxy]-3,4-dihydro-2*H*-chromen-4-yl}-piperazin, seine Enantiomeren sowie seine Additionssalze mit einer pharmazeutisch annehmbaren Säure und
- trans-1-{3-(3-Chlor-5-fluorbenzyloxy)-3,4-dihydro-2H-chromen-4-yl}-piperazin, seine Enantiomeren sowie seine Additionssalze mit einer pharmazeutisch annehmbaren Säure,

**8.** Verfahren zur Herstellung der Verbindungen der Formel (I) nach Anspruch 1, ausgehend von der Verbindung der Formel (Va) in der relativen trans-Konfiguration:

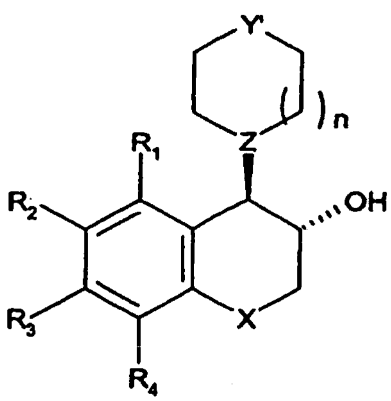

(Va)

in der $R_1$, $R_2$, $R_3$, $R_4$, X, n und Z die oben angegebenen Bedeutungen besitzen und Y' ein Sauerstoffatom oder eine Gruppe ausgewählt aus $NP_1$ und $CHR'_8$ bedeutet, worin $R'_8$ ein Wasserstoffatom oder eine Gruppe NHP, darstellt und $P_1$ eine Schutzgruppe für die Aminfunktion bedeutet,

- welche Verbindung der Formel (Va) man, wenn man die Verbindungen der Formel (I) in der relativen trans-Konfiguration herstellen will, mit einer Verbindung der Formel (VI) umsetzt:

G-Ak-Ar (VI)

in der Ak und Ar die bezüglich der Formel (I) angegebenen Bedeutungen besitzen und G eine austretende Gruppe bedeutet, wie beispielsweise ein Halogenatom oder eine p-Toluolsulfonat-, Trifluormethansulfonat- oder Methansulfonat-gruppe bedeutet,
zur Bildung der Verbindung der Formel (VIIa) in der relativen trans-Konfiguration:

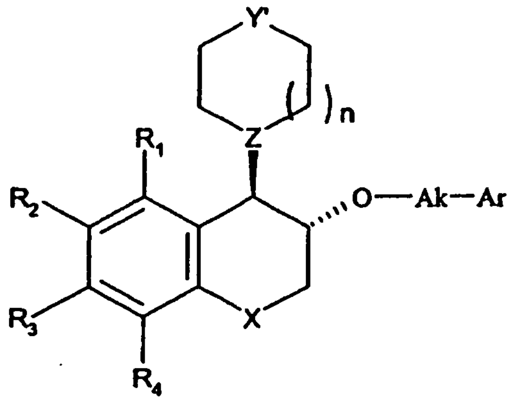

(VIIa)

in der $R_1$, $R_2$, $R_3$, $R_4$, X, n, Y', Z, Ak und Ar die oben angegebenen Bedeutungen besitzen,
von der man, wenn Y' eine Schutzgruppe $P_1$, wie sie oben definiert worden ist, trägt, diese Schutzgruppe abspaltet und die man dann, wenn man die Verbindungen herstellen will, bei denen Y eine Gruppe $NR_7$ darstellt, worin $R_7$ von einem Wasserstoffatom verschieden ist, alkyliert,
zur Bildung der Verbindungen der Formel (Ia), einem Sonderfall der Verbindungen der Formel (I), die in der relativen trans-Konfiguration vorliegen:

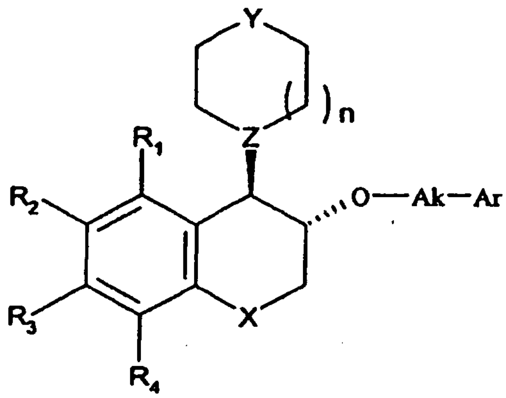

(Ia)

in der $R_1$, $R_2$, $R_3$, $R_4$, X, n, Y, Z, Ak und Ar die bezüglich der Formel (I) angegebenen Bedeutungen besitzen,
- oder man oxidiert die Verbindung der Formel (Va), wenn man die Verbindungen der Formel (I) in der relativen cis-Konfiguration herstellen will,

zur Bildung der racemischen Verbindung der Formel (VIII):

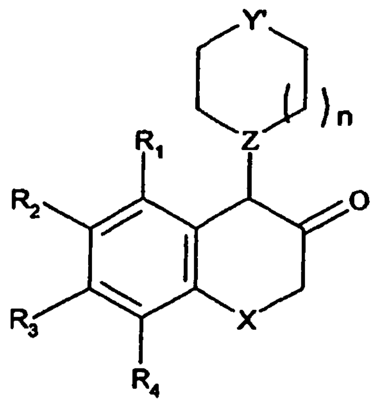

(VIII)

in der $R_1$, $R_2$, $R_3$, $R_4$, X, Z, n und Y' die oben angegebenen Bedeutungen besitzen, welche zu dem entsprechenden Alkohol reduziert wird, dessen Diastereoisomeren man trennt und das Isomere der Formel (Vb) in der relativen cis-Konfiguration isoliert:

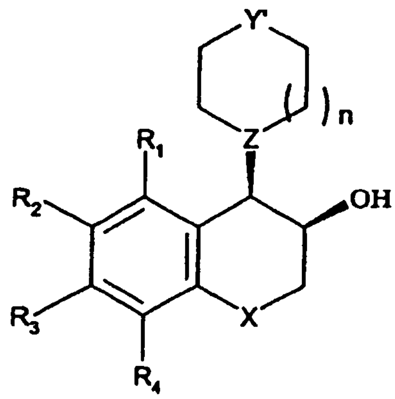

(Vb)

in der $R_1$, $R_2$, $R_3$, $R_4$, X, Y', Z und n die oben angegebenen Bedeutungen besitzen, welches man mit einer Verbindung der Formel (VI). wie sie oben definiert worden ist, umsetzt zur Bildung der Verbindung der Formel (VIIb) in der relativen cis-Konfiguration:

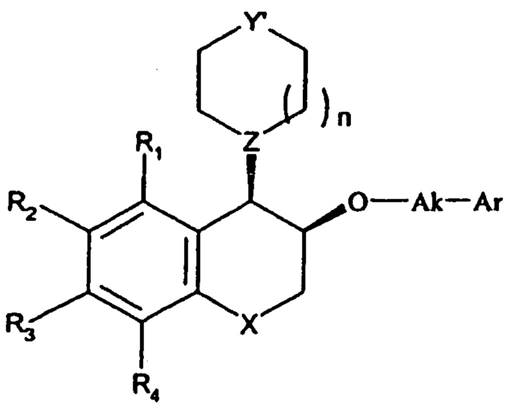

(VIIb)

in der $R_1$, $R_2$, $R_3$, $R_4$, X, n, Y', Z, Ak und Ar die oben angegebenen Bedeutungen besitzen,
von der man, wenn Y' eine Schutzgruppe $P_1$, wie sie oben definiert worden ist, trägt, diese Schutzgruppe abspaltet und die man dann, wenn man die Verbindungen herstellen will, bei denen Y eine Gruppe $NR_7$ darstellt, worin $R_7$ von einem Wasserstoffatom verschieden ist, alkyliert,

zur Bildung der Verbindungen der Formel (Ib), einem Sonderfall der Verbindungen der Formel (I), die in der relativen cis-Konfiguration vorliegen:

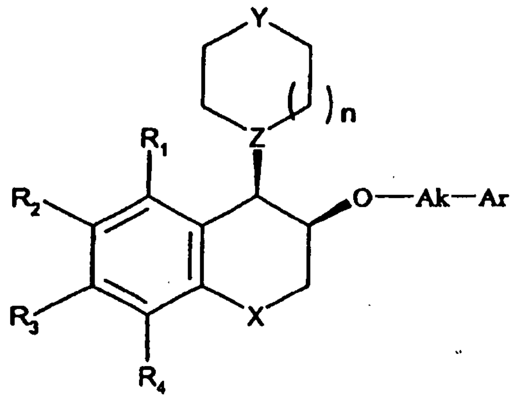

(Ib)

in der $R_1$, $R_2$, $R_3$, $R_4$, X, Y, Z, n, Ak und Ar die bezüglich der Formel (I) angegebenen Bedeutungen besitzen, welche Verbindungen der Formeln (Ia) und (Ib) man gewünschtenfalls mit Hilfe einer klassischen Reinigungsmethode reinigt, man gewünschtenfalls in ihre optischen Isomeren auftrennt und gewünschtenfalls in ihre Additionssalze mit einer pharmazeutisch annehmbaren Säure überführt.

**9.** Pharmazeutische Zubereitung, enthaltend als Wirkstoff eine Verbindung nach einem der Ansprüche 1 bis 7 in Kombination mit einem oder mehreren inerten, nichttoxischen und pharmazeutisch annehmbaren Hilfsstoffen.

**10.** Verwendung der Verbindungen der Formel (I) nach einem der Ansprüche 1 bis 7 für die Herstellung von Arzneimitteln, die als Inhibitoren der Wiederaufnahme von Serotonin nützlich sind.

**11.** Verwendung der Verbindungen der Formel (I) nach einem der Ansprüche 1 bis 7 für die Herstellung von Arzneimitteln, die als Inhibitoren der Wiederaufnahme von Serotonin und als $NK_1$-Antagonisten nützlich sind.

**12.** Verwendung der Verbindungen der Formel (I) nach einem der Ansprüche 1 bis 7 für die Herstellung von Arzneimitteln zur Behandlung von Depressionszuständen, Angstzuständen, impulsiven Störungen, aggressivem Verhalten, Drogenmißbrauch, Fettsucht, Appetitstörungen, Schmerzen, Entzündungszuständen, Demenzien, psychotischen Zuständen, Störungen der chrono-biologischen Rhythmen, Übelkeit und Magen-Darm-Störungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6136824 A
- GR 205171


### Littérature non-brevet citée dans la description


- **GOODNICK ; GOLDSTEIN.** *J Psychopharmacol,* 1998, vol. 12 (B), S55-S87
- **CHEER ; GOA.** *Drugs,* 2001, vol. 61, 81-110
- **MACQUEEN et al.** *CNS Drug Rev,* 2001, vol. 7, 1-24
- **WAGSTAFF et al.** *Drugs,* 2002, vol. 62, 655-703
- **FEIGHNER.** *J Clin Psychiatry,* 1999, vol. 60 (22), 18-22
- **BAKKER et al.** *t clin Psychopharmacol,* 2000, vol. 15 (2), S25-S30
- **DAVIDSON.** *Int Clin Psychopharmacol,* 2000, vol. 15 (1), S13-S17
- **SCHNEIER.** *J Clin Psychiatry,* 2001, vol. 62 (1), 43-48
- **MARONA-LEWICKA ; NICHOLS.** *Stress,* 1997, vol. 2, 91-100
- **MAR et al.** *Pharmacol Biochem Behav,* 2002, vol. 73, 703-712
- **WILL et al.** *Mol Psychiatry,* 2003, vol. 8, 925-932
- **BALLENGER.** *J Clin Psychiatry,* 2004, vol. 65, 1696-1707
- **MALBERG ; DUMAN.** *Neuropsychopharmacology,* 2003, vol. 28, 1562-1571
- **SANTARELLI et al.** *Science,* 2003, vol. 301, 805-809
- **CZEH et al.** *Neuropsychopharmacology,* 2005, vol. 30, 67-79
- **MALBERG ; SCHECHTER.** *Curr Pharm Des,* 2005, vol. 11, 145-155
- **NJUNG'E ; HANDLEY.** *Br J Pharmacol,* 1991, vol. 104, 105-112
- **ICHIMARU et al.** *Jpn J Pharmacol,* 1995, vol. 68, 65-70
- **PIGOTT ; SEAY.** *J Clin Psychiatry,* 1999, vol. 60, 101-106
- **VYTHILINGUM et al.** *Int Clin Psychopharmacol,* 2000, vol. 15 (2), S7-S13
- **KNUTSON et al.** *Am J Psychiatry,* 1998, vol. 155, 373-379
- **LANCTOT et al.** *Neuropsychopharmacology,* 2002, vol. 27, 646-654
- **NEW et al.** *Psychopharmacology,* vol. 2004 (176), 451-458
- **PROIETTO et al.** *Expert Opin Investig Drugs,* 2000, vol. 9, 1317-1326
- **LJUNG et al.** *J Intern Med,* 2001, vol. 250, 219-224
- **APPOLINARIO et al.** *CNS Drugs,* 2004, vol. 18, 629-651
- **APPOLINARIO ; MCELROY.** *Curr Drug Targets,* 2004, vol. 5, 301-307
- **ARAGONA et al.** *Eur J Pain,* 2005, vol. 9, 33-38
- **MILLAN et al.** *Neuropharmacology,* 2002, vol. 42, 677-684
- **DUMAN et al.** *J Pharmacol Sci,* 2004, vol. 94, 161-165
- **OTSUKA et al.** *J Anesth,* 2004, vol. 15, 154-158
- **LYKETOS et al.** *Am JPsychiatry,* 2000, vol. 157, 1686-1689
- **LANCTOT et al.** *J Neuropsyhiatry Clin Neurosci,* 2001, vol. 13, 5-21
- **POLLOCK et al.** *Am J Psychiatry,* 2002, vol. 159, 460-465
- **RUPNIAK et al.** *Behav Pharmacol,* 2001, vol. 12, 497-508
- **RUPNIAK et al.** *Neuropharmacology,* 2003, vol. 44, 516-523
- **KRAMER et al.** *Neuropsychopharmacology,* 2004, vol. 29, 385-392
- **DABLEH et al.** *Eur J Pharmacol,* 2005, vol. 507, 99-105
- **SANTARELLI et al.** *Proc Natl Acad Sci USA,* 2001, vol. 98, 1912-1927
- **VARTY et al.** *Neuropsychopharmacology,* 2002, vol. 27, 371-379
- **RUPNIAK ; KRAMER.** *Neuropsychopharmacology,* 2002, vol. 13, 169-177
- **BALLARD et al.** *Eur J Pharmacol,* 2001, vol. 412, 255-264
- **SPOOREN et al.** *Eur J Pharmacol,* 2002, vol. 435, 161-170
- **STEINBERG et al.** *J Pharmacol Exp Ther,* 2002, vol. 303, 1180-1188

- **VAN DER HART et al.** *Mol Psychiatry,* 2002, vol. 7, 933-941
- **MORCUENDE et al.** *Eur J Neurosci,* 2003, vol. 18, 1828-1836
- **GUEST et al.** *Brain Res,* 2004, vol. 1002, 1-10
- **CULMAN et al.** *Br J Pharmacol,* 1995, vol. 114, 1310-1316
- **TSCHÖPE et al.** *Br J Pharmacol,* 1992, vol. 107, 750-755
- **SIEGEL ; SCHUBERT.** *Rev Neurosci,* 1995, vol. 6, 47-61
- **DE FELIPE et al.** *Nature,* 1998, vol. 392, 394-397
- **MURTRA et al.** *Nature,* 2000, vol. 405, 180-183
- **RIPLEY et al.** *Neuropharmacology,* 2002, vol. 43, 1258-1268
- **GADD et al.** *J Neurosci,* 2003, vol. 23, 8271-8280
- **ZACHRISSON et al.** *Eur Neuropsychopharmacol,* 2000, vol. 10, 355-363
- **ANDERSON et al.** *J Pharmacol Exp Ther,* 1995, vol. 274, 928-936
- **PRIEST et al.** *Brain Res Mol Brain Res,* 1995, vol. 28, 61-71
- **DANIELS et al.** *Neurosci Lett,* 2003, vol. 338, 111-114
- **KRAMER et al.** *Science,* 1998, vol. 281, 1640-1644
- **SHIBATA et al.** *Brain Res,* 1992, vol. 597, 257-263
- **CHALLET et al.** *Brain Res,* 1998, vol. 800, 32-39
- **CHALLET et al.** *Neuropharmacology,* 2001, vol. 40, 408-415
- **GANNON et al.** *Neuropharmacology*
- **SEGUIN et al.** *Pain,* 1995, vol. 61, 325-343
- **SANGER.** *Br J Pharmacol,* 2004, vol. 141, 1303-1312
- **SEABROOK et al.** *Eur J Pharmacol,* 1996, vol. 317, 129-135
- **HOLZER.** *Digestion,* 1998, vol. 59, 269-283
- **JOOS ; PAUWELS.** *Curr Opin Pharmacol,* 2001, vol. 1, 235-241
- **MCALLISTER ; PRATT.** *Eur J Pharmacol,* 1998, vol. 353, 141-148
- **GARDNER et al.** *Regulatory Peptides,* 1996, vol. 65, 45-53
- **PATEL ; LINDLEY.** *Expert Opin. Pharmacother,* 2003, vol. 4, 2279-2296
- **RAFFA.** *Neurosci Biobehav Rev,* 1998, vol. 22, 789-813
- **GUIARD et al.** *J Neurochem,* 2004, vol. 89, 54-63
- **FROGER et al.** *J Neurosci,* 2001, vol. 21, 8188-8197
- **BAGDY et al.** *Int J Neuropsychopharmacol,* 2001, vol. 4, 399-408
- **GOLDSTEIN ; GOODNICK.** *J Psychopharmacol,* 1998, vol. 12 (B), S55-S87
- **EDWARDS ; ANDERSON.** *Drugs,* 1999, vol. 57, 507-533
- **WAUGH ; GOA.** *CNS Drugs,* 2003, vol. 17, 343-362
- **MONTGOMERY et al.** *J Affect disord,* 2002, vol. 69, 119-140
- **HIRSCHFELD, J.** *Clin Psychiatry,* 2003, vol. 64 (18), 20-24
- *La publication Bioorganic & Medicinal Chemistry Letters,* 2002, vol. 12 (21), 3195-3198
- *La publication Indian Journal of Chemistry,* 2000, vol. 39B, 468-471
- *Synth. Comm.,* 1994, 2777
- *J. Org. Chem.,* 1969, vol. 34 (1), 207
- *J. Org. Chem.,* 1998, vol. 63, 864
- *Tetrahedron Letters,* 2000, vol. 41, 8661